# EUROPÄISCHE PATENTSCHRIFT

(11) **EP 3 421 063 B1**
(45) Veröffentlichungstag und Bekanntmachung des Hinweises auf die Patenterteilung: **17.03.2021**
(21) Anmeldenummer: 18184410.1
(22) Anmeldetag: 14.04.2010
(51) Int. Cl.: A61M 1/36, A61M 1/34, A61M 39/00, F16K 15/14

(54) **EXTERNE FUNKTIONSEINRICHTUNG UND BEHANDLUNGSVORRICHTUNG**
EXTERNAL FUNCTIONAL DEVICE AND TREATMENT DEVICE
DISPOSITIF FONCTIONNEL EXTERNE ET DISPOSITIF DE TRAITEMENT

(30) Priorität: 23.04.2009 DE 102009018664; 10.06.2009 DE 102009024469; 10.06.2009 US 18560309 P
(43) Veröffentlichungstag der Anmeldung: 02.01.2019
(62) Teilanmeldung aus: 10713589.9
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB

(56) Entgegenhaltungen:
- EP-A2- 1 953 432
- WO-A1-2005/116497
- US-A- 3 941 149
- US-A- 4 815 705
- US-B1- 6 537 258

## Beschreibung

Die vorliegende Erfindung betrifft eine externe Funktionseinrichtung mit einer Ventilvorrichtung. Sie betrifft ferner eine Behandlungsvorrichtung.

Aus Kosten- und Hygienegründen werden in technischen Vorrichtungen, wie beispielsweise medizintechnischen Behandlungsvorrichtungen, labortechnischen Vorrichtungen oder auch Vorrichtungen zur Nahrungsmittel- oder Arzneimittelherstellung oftmals externe Funktionseinrichtungen als Einmalteilsysteme, wie beispielsweise Disposable-Kassetten, eingesetzt.

Derartige Einmalteilsysteme können Kanäle und Kammern zum gezielten Führen von Flüssigkeiten und Gasen sowie Vorrichtungen, wie Ventilvorrichtungen, zum Variieren oder Steuern des Durchflusses dieser Fluide aufweisen. In der EP 1 953 432 A2 ist eine derartige Ventilvorrichtung zum Steuern eines Fluidflusses mittels Fluiddrucks offenbart.

Aufgabe der vorliegenden Erfindung ist es, eine weitere externe Funktionseinrichtung und eine entsprechende Behandlungsvorrichtung vorzuschlagen.

Die erfindungsgemäße Aufgabe wird durch eine externe Funktionseinrichtung wie hierin beschrieben gelöst.

Die erfindungsgemäße externe Funktionseinrichtung weist alle Merkmale des Anspruchs 1 auf.

Die erfindungsgemäße externe Funktionseinrichtung weist wenigstens eine Ventilvorrichtung auf, welche wenigstens einen in wenigstens einem Abschnitt hiervon elastischen Ventileinsatz und wenigstens eine Aufnahmeeinrichtung für den Ventileinsatz aufweist.

Der Ventileinsatz der offenbarten Ventilvorrichtung ist derart vorgesehen und ausgestaltet, dass er bei Wegeinprägung und/oder Kraftaufbringung auf den Ventileinsatz oder auf einen anderen Abschnitt der Ventilvorrichtung zwischen wenigstens drei unterschiedlichen Ventilzuständen schaltbar oder wechselbar ist. Unter der "Schaltbarkeit" des Ventileinsatzes wird im Folgenden die Möglichkeit im weitesten Sinne verstanden, mittels des Ventileinsatzes wenigstens drei Ventilzustände zu erzielen.

Unter einer "Wegeinprägung" wird erfindungsgemäß vorzugsweise eine Veränderung einer variierbaren Abmessung einer Struktur oder eines Bauteils verstanden. So kann eine Wegeinprägung bei einer Spiralfeder deren Längung oder deren Stauchung bedeuten. Bezogen auf den Ventileinsatz der offenbarten Ventilvorrichtung bedeutet eine Wegeinprägung dessen Stauchung, Streckung, Verformung und dgl.

Der Begriff "Ventileinsatz", wie er hierin verwendet wird, bezeichnet den aufgrund seiner Elastizität verformbaren Grundkörper der offenbarten Ventilvorrichtung. Der Ventileinsatz ist in seinem Gebrauch vom Fluid ganz oder teilweise umströmt, zumindest aber bei Vorliegen eines Fluids an der Ventilvorrichtung mit Fluid in Kontakt.

Eine "Aufnahmeeinrichtung" für den Ventileinsatz, wie sie hierin verwendet wird, bezeichnet eine zum Aufnehmen des Ventileinsatzes ausgestaltete und/oder vorgesehene Aufnahmeeinrichtung.

Das "Aufnehmen" kann ein Umfassen, ein Abstützen, ein Herstellen eines Funktionszusammenhangs zwischen Aufnahmeeinrichtung und Ventileinsatz bewirken, und dergleichen.

Bevorzugte Ausführungsformen sind jeweils Gegenstand der Unteransprüche. Ferner sind im Folgenden Merkmale aufgeführt, welche Merkmale der offenbarten Ventilvorrichtung oder ihres Ventileinsatzes sein können. Sie können in jeder Ausführungsform unabhängig von anderen Merkmalen verwirklicht sein.

So kann die offenbarte Aufnahmeeinrichtung eine zum Aufnehmen des Ventileinsatzes geeignete Öffnung aufweisen.

Die Schaltbarkeit des Ventileinsatzes zwischen wenigstens drei unterschiedlichen Ventilzuständen kann der Ventileinsatz insbesondere seiner Elastizität verdanken.

Der offenbarte Ventileinsatz kann ausgestaltet sein, um allein durch seine elastische Verformung oder elastische Rückstellung oder elastische Verformbarkeit in verschiedene Ventilzustände überzugehen oder diese zu ermöglichen.

Eine Veränderung der Lage des Ventilabschnitts oder eines Abschnitts hiervon bezogen auf beispielsweise einen Abschnitt der Ventilvorrichtung wie der Aufnahmeeinrichtung, welcher kein Abschnitt des Ventilabschnitts ist, kann zum Schalten zwischen den Ventilzuständen entbehrlich sein.

Der Ventileinsatz kann sich aufgrund der Kraft elastisch - d. h. reversibel - verformen, beispielsweise krümmen, falten verbiegen oder dergleichen. Je nach Wegeinprägung und/oder Kraftaufbringung auf den Ventileinsatz kann dieser somit verschiedene Ventilzustände einnehmen. Die Rückstellkräfte aufgrund der elastischen Verformungen können Funktionen wie Schutz gegen Herausfallen, Selbstrückstellung, Dichtung und/oder Ansprechdruck bewirken.

Der Ventileinsatz kann symmetrisch sein. Er kann eine rotationssymmetrische Gestalt haben.

Die offenbarte Aufnahmeeinrichtung kann ausgelegt und/oder vorgesehen sein, um den Ventileinsatz im Wesentlichen passgenau aufzunehmen und/oder unter Einbau-Vorspannung zu halten.

Sie kann derart ausgelegt und/oder vorgesehen sein, um Toleranzen beim Aufbauen und/oder Verpressen des Ventileinsatzes zuzulassen.

Die Aufnahmeinrichtung kann aus einem oder mehreren unelastischen Materialien, wie beispielsweise duroplastischen Kunststoffen, Metallen und dergleichen sowie Kombinationen hergestellt sein.

Die Steifigkeit der Aufnahmeeinrichtung kann vorzugsweise wesentlich höher als die des Ventileinsatzes sein. Die Biegesteifigkeit und/oder die Kompressibilität des Ventileinsatzes können beispielsweise 60 N/mm² bis 300 N/mm² betragen, und jene der Aufnahmeeinrichtung können beispielsweise 800 N/mm² bis 2400 N/mm² betragen.

Die Aufnahmeeinrichtung ist in einem Gehäuseelement, wie beispielsweise einem Hartteil, der externen Funktionseinrichtung angeordnet, welche beispielsweise als Disposable-Kassette ausgestaltet ist.

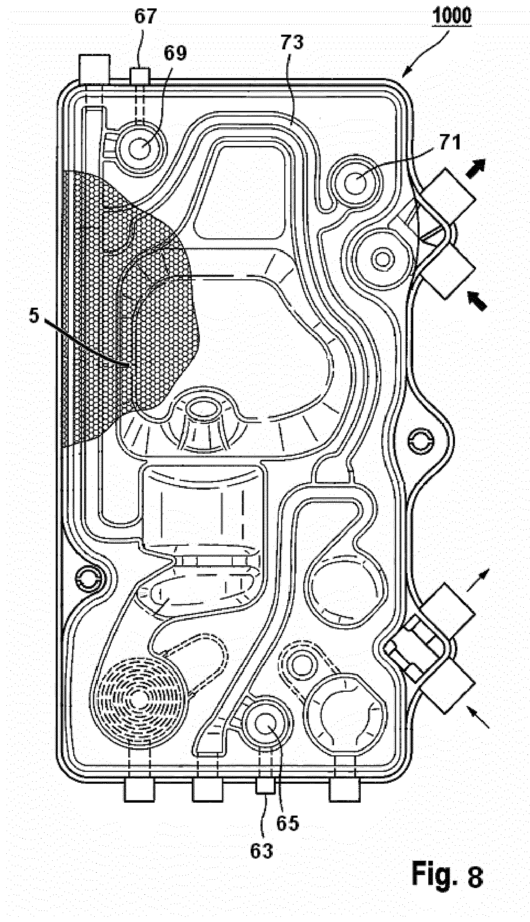

Die "externe Funktionseinrichtung" ist als Fluidbehandlungskassette ausgestaltet. Eine Fluidbehandlungskassette, Blutbehandlungskassette oder Disposable-Kassette im Sinne der vorliegenden Erfindung ist beispielsweise in der von der Anmelderin der vorliegenden Erfindung in den beim DPMA am 23. April 2009 und 10. Juni 2009 eingereichten Anmeldungen als 10 2009 018 664.6 und mit jeweils dem Titel *"Externe Funktionseinrichtung, Blutbehandlungsvorrichtung zum Aufnehmen einer erfindungsgemäßen externen Funktionseinrichtung, sowie Verfahren"* beschrieben. Eine bevorzugte Ausführungsform der darin offenbarten Blutkassette ist in den Fig. 8, 9 und 10 der vorliegenden Anmeldung wiedergegeben.

Der Begriff "Gehäuseelement", wie er hierin verwendet wird, bezeichnet ein Bauelement, das Teil einer externen Funktionseinrichtung, einer Behandlungsvorrichtung und/oder sowohl der externen Funktionseinrichtung als auch der Behandlungsvorrichtung sein kann. Das Gehäuseelement kann aus einem starren, d. h. im Wesentlichen unelastischen, Material gebildet sein.

Das Gehäuseelement einer externen Funktionseinrichtung kann verschiedene Funktionen ausfüllen. Es kann, ohne darauf beschränkt zu sein, einen Fluidkanal darstellen, einen Dichtsitz des Ventileinsatzes, z. B. als Trennung zwischen einem Einströmungskanal und einem Ausströmungskanal für Fluide, welche die Ventilvorrichtung durchströmen, wie Blut oder Substituatflüssigkeit oder ein Sterilisierfluid (eine Flüssigkeit und/oder ein Gas) oder eine Spülflüssigkeit, darstellen.

Das Gehäuseelement kann als eine Halterung für den Ventileinsatz dienen, eine korrekte axiale Verschiebung oder Verpressung des Ventileinsatzes gewährleisten und dergleichen. Die unbestimmten Artikel wie "ein" oder "einen" sind hierbei - wie in der gesamten Anmeldung - nicht beschränkend und nicht als Zahlwörter zu verstehen.

Das Gehäuseelement kann Kanäle, wie Ein- und Ausströmungskanäle, durch welche Fluide in die Ventilvorrichtung eingeleitet bzw. aus dieser ausgetragen werden, aufweisen. Solche Kanäle können als halboffene oder als geschlossene Kanäle gestaltet sein. Sie können beispielsweise auch in einem Abschnitt als halboffene Kanäle und in einem anderen Abschnitt als geschlossene Kanäle ausgestaltet sein.

Geschlossene Kanäle können sich besonders als Übergang zu unmittelbar angeschlossenen Konnektorelementen, wie beispielsweise Luer-Stutzen oder Schlauchmuffen, eignen.

Halboffene Kanäle können insbesondere in externen Funktionseinrichtungen, wie beispielsweise einer Disposable-Kassette, bevorzugt sein. Die Verwendung halboffener Kanäle in einer externen Funktionseinrichtung kann eine große Gestaltungsfreiheit beim "Fluidlayout" zulassen, beispielsweise durch Ausgestalten von Führungen oder Verzweigungen hin zu Mess- und/oder Entlüftungskammern.

Im Gehäuseelement vorgesehene Kanäle, zum Beispiel in die Ventilvorrichtung hineinführende Einströmungskanäle und/oder aus der Ventilvorrichtung herausführende Ausströmungskanäle, können in einem beliebigen Winkel zueinander ausgeführt sein. Dies kann ein Winkel von genau oder etwa 180 Grad oder auch ein Winkel von weniger als 90 Grad sein. Bevorzugt kann der Winkel einen Wert in einem Bereich von 45 Grad bis 315 Grad annehmen.

Auch eine koaxiale Anordnung der Kanäle zu einer ggf. vorhandenen Symmetrieachse des Ventileinsatzes ist ausführbar. Eine derartige Anordnung kann bevorzugt sein. Dies gilt insbesondere dann, wenn der Ventileinsatz zum Beispiel nicht in einer externen Funktionseinrichtung, sondern als sogenanntes Inline-Funktionselement in eine Schlauchleitung geschaltet bzw. eingefügt wird.

Sollen beispielsweise beide Strömungskanäle koaxial zum Ventileinsatz ausgerichtet werden, so können in den Sitzbereich des Gehäuseelements Unterbrechungen eingearbeitet sein, welche ein axiales Umfließen des Ventileinsatzes durch die, die Ventilvorrichtung durchströmenden, Fluide zulassen.

In einem solchen Fall sind bevorzugt Durchbrüche in einem Biegeringbereich des Ventileinsatzes vorgesehen. Dabei können Gehäuseelemente auf besonders geringem Bauraum, z. B. mit kleinem Durchmesser, ausgestaltet sein. Die Ventilvorrichtung kann insgesamt besonders strömungsgünstig ausgeführt werden.

Der Ventileinsatz kann durch gestufte Wegeinprägung und/oder Kraftaufbringung, zum Beispiel mittels eines Aktors auf eine Oberseite des Ventileinsatzes, in verschiedene, definierte Verformungszustände gebracht werden. Je nach der auf den Ventileinsatz aufgebrachten Kraft und/oder der Wegeinprägung können bestimmte Ventilfunktionen oder Ventilzustände oder Ventilstellungen realisiert werden. Der Ventileinsatz kann in einigen oder allen dieser Ventilzustände unter einer elastischen Spannung stehen.

Ein Ventilzustand kann ein geöffneter bzw. ein offener Ventilzustand sein.

In einem geöffneten bzw. offenen Ventilzustand befindet sich der Ventileinsatz bzw. die Ventilvorrichtung dann, wenn keine äußere Kraft auf den Ventileinsatz aufgebracht wird. Der Ventileinsatz kann dabei in die Aufnahmeeinrichtung eingelegt sein. Der Ventileinsatz kann in diesem Zustand frei von Vorspannungen und/oder zwischen Bauelementen erzeugten Verspannungen sein.

Der Durchgang von Fluiden kann in diesem Zustand maximal sein. Durchlassöffnungen für Fluide, welche den Ventileinsatz durchströmen, können in diesem Zustand maximal geöffnet sein.

Ein Ventilzustand kann ein Sterilisationszustand sein.

Zum Sterilisieren der Ventilvorrichtung oder der externen Funktionseinrichtung mit der offenbarten Ventilvorrichtung liegt der Ventileinsatz bevorzugt im Sterilisationszustand vor.

Im Sterilisationszustand kann der Ventileinsatz bevorzugt die gleiche Konfiguration oder Gestalt wie im geöffneten Ventilzustand aufweisen oder einnehmen.

Der Ventileinsatz kann im Sterilisationszustand wiederum frei von äußeren Kräften und/oder Vorspannungen und/oder Verspannungen mit weiteren Bauelementen, wie beispielsweise der Aufnahmeeinrichtung, und/oder frei von Wegeinprägungen sein.

Im Sterilisationszustand kann ein bidirektionaler Durchfluss von Fluiden durch die Ventilvorrichtung möglich sein.

Im Sterilisationszustand kann das Sterilisationsmittel vorzugsweise alle bautechnisch zugänglichen Volumen und Teilvolumen, Öffnungen, Hinterschneidungen und dergleichen der Ventilvorrichtung erreichen.

Ein weiterer Ventilzustand kann ein Rückschlag-Ventilzustand sein.

Ein Rückschlagventil ist aus dem Stand der Technik beispielsweise als ein Verschlusskörper in Kugel- oder Zylinderform in Verbindung mit vorgespannten Federn bekannt. Ein Rückschlagventil kann ebenso als Druckbegrenzer wirken.

Der "Rückschlagventilzustand" des Ventileinsatzes bzw. der Ventilvorrichtung der vorliegenden Erfindung kann zur Richtungsbestimmung der Strömung eines Fluids innerhalb eines Strömungskanals - oder mehrerer - dienen.

Im Rückschlagventilzustand kann der Durchgang eines Fluids in einer Strömungsrichtung gesperrt sein.

Ein weiterer Ventilzustand kann ein Steuer- oder Regel-Ventilzustand sein.

Im Steuer- oder Regel-Ventilzustand ist auf den Ventileinsatz der Ventilvorrichtung eine Kraft -vorzugsweise variierbar - aufgebracht, mittels welcher eine Durchflussmenge der Fluide, welche die Ventilvorrichtung durchströmen, steuer- oder regelbar ist.

Die Ventilvorrichtung kann dazu beispielsweise aktiv durch kraft- und/oder bewegungsübertragende Aktoren angesteuert werden.

Geeignete Aktoren können beispielsweise an eine Behandlungsvorrichtung angekoppelt und insbesondere von dieser betätigt oder gesteuert/geregelt werden.

Die absolut auf den Ventileinsatz aufzubringende Kraft und/oder die aufzubringende Wegeinprägung zum Steuern oder Regeln, zum Beispiel zum Einstellen einer bestimmten oder definierten Durchflussmenge, kann in Abhängigkeit von der erwünschten oder erforderlichen Durchflussmenge der Fluide, welche die Ventilvorrichtung durchströmen, festgelegt sein.

Sie kann beispielsweise in einer Steuereinrichtung, einer CPU oder dergleichen vorprogrammiert sein.

Die Kraft und/oder die Wegeinprägung (Verschiebung) können durch Aktoren aufgebracht werden, zum Beispiel auf die Oberseite des Ventileinsatzes.

Ein weiterer, möglicher Ventilzustand ist ein geschlossener Ventilzustand.

Im geschlossenen Ventilzustand kann die Durchflussmenge der die Ventilvorrichtung durchströmenden Fluide derart verringert sein, dass kein oder im Wesentlichen kein Durchfluss stattfindet.

Der Ventileinsatz kann derart angeordnet sein, dass die zum Durchtritt von Fluiden vorgesehenen Öffnungen im Wesentlichen unzugänglich oder verschlossen sind. Die Öffnungen können vollständig unzugänglich oder verschlossen sein.

Der Ventilzustand kann ein permanent geschlossener Ventilzustand sein.

Der Ventileinsatz kann ganz oder wenigstens in Abschnitten hiervon rotationssymmetrisch ausgestaltet sein.

In einer weiter bevorzugten Ausführungsform weist der Ventileinsatz in wenigstens einem Abschnitt ein elastomeres Material auf. Der Ventileinsatz kann ganz oder wenigstens in Abschnitten hiervon elastisch ausgestaltet sein.

Zu den geeigneten elastomeren Materialien zählen, ohne darauf beschränkt zu sein, Gummi, Silikon, TPE (Thermoplastische Elastomere), TPE-U (teils auch mit TPU abgekürzt); Thermoplastisch Elastomere auf Basis von Polyurethan), Kunststoff, PVC und dergleichen mehr.

Flüssigkristalline Elastomere und/oder thermoplastische Elastomere können zu den geeigneten elastomeren Materialien zählen.

Thermoplastische Elastomere können hierbei vorzugsweise Mischungen aus einem oder mehreren Elastomeren und einem oder mehreren Thermoplasten sein.

Solche Thermoplaste können zum Beispiel zum Herstellen starrer Gehäusekomponenten einer externen Funktionseinrichtung geeignet sein.

Die thermoplastischen Elastomere können mit gespritzten Gehäuseelementen verklebt und/oder verschweißt und/oder in anderer Weise kraft- und/oder form- und/oder stoffschlüssig verbunden sein.

Der Ventileinsatz kann Einprägungen im Sinne von Abschnitten dünneren oder weicheren Materials bezogen auf andere Abschnitte hiervon aufweisen, welche eine besondere Elastizität oder Verformbarkeit desselben gewährleisten können. Derartige Einprägungen können durch Druck mittels Walzen, Rollen, Stempel und dergleichen, aber auch bei einem Gießvorgang oder Extrusionsvorgang ausgestaltet werden.

Der Ventileinsatz kann ein vorbestimmtes Faltungsmuster oder einen faltigen oder gefalteten Abschnitt aufweisen, welcher nach einer Streckung elastisch in einen Grundzustand rückführbar ist.

Der Ventileinsatz kann als ein Einkomponenten-Ventilsatz, aber auch als Mehrkomponenten-Ventileinsatz ausgestaltet sein.

Der Ventileinsatz weist wenigstes eine erste Abdichtungseinrichtung auf, die vorgesehen und ausgestaltet ist, um in wenigstens einem Abschnitt hiervon mit wenigstens einem Abschnitt einer zweiten Abdichtungseinrichtung der Aufnahmeeinrichtung form- und/oder kraftschlüssig verpressbar zu sein.

Die "erste Abdichtungseinrichtung" ist ein elastischer Dichtring. Sie kann eine umlaufende, insbesondere geschlossene, Dichtlippe sein. Die Dichtlippe kann z. B. eine ebene Form, eine konische Form und/oder eine leicht gewellte Form aufweisen. Die erste Abdichtungseinrichtung kann je nach Verformungszustand des Ventileinsatzes an einer definierten Dichtfläche an der Aufnahmeeinrichtung, zum Beispiel der zweiten Abdichtungseinrichtung, oder am Ventileinsatz aufliegen.

Die erste Abdichtungseinrichtung kann eine Scheibenform aufweisen oder scheibenförmig sein. Die erste Abdichtungseinrichtung kann über ein axiales Zentrum, zum Beispiel in einem zentralen Bereich einer scheibenförmigen ersten Abdichtungseinrichtung, vorgespannt sein. Beim zentralen Bereich kann es sich um einen inneren Bereich handeln. Der zentrale Bereich ist vorzugsweise ein Bereich einer Materialanhäufung oder eines "Kerns".

Die "zweite Abdichtungseinrichtung" kann ein starrer Dichtring sein.

Die zweite Abdichtungseinrichtung kann am Ventileinsatz oder an der Aufnahmeeinrichtung, ferner auch an einer anderen Einrichtung vorgesehen sein.

Die zweite Abdichtungseinrichtung kann eine leicht wellige Form, aber auch eine ebene Form aufweisen.

Durch die axiale Vorspannung der ersten Abdichtungseinrichtung des Ventileinsatzes kann eine Angleichung der ersten Abdichtungseinrichtung an die Form der zweiten Abdichtungseinrichtung erfolgen.

Eine solche Angleichung kann bevorzugt eine Dichtwirkung erzeugen.

Diese Dichtwirkung kann in einem Rückschlagventilzustand durch den Fluiddruck weiter verstärkt werden. Die erste Abdichtungseinrichtung kann verstärkt an die zweite Abdichtungseinrichtung angepresst werden. Ebenso kann die eventuell von der ideal ebenen Form abweichende Gestalt der zweiten und/oder der ersten Abdichtungseinrichtung mittels einer auf die elastische Scheibe der ersten Abdichtungseinrichtung wirkende Flächenlast an deren Form angeglichen werden.

Die erste Abdichtungseinrichtung und die zweite Abdichtungseinrichtung können einen nötigen geometrischen Toleranzausgleich gewährleisten.

Ein derartiger Toleranzausgleich kann über die Fähigkeit zu einer winkligen und/oder schüsselförmigen Verbiegung der ersten Abdichtungseinrichtung unter dem Einfluss von Vorspannung und/oder Fluiddruck wirken.

Ein geometrischer Toleranzausgleich kann auch bei einer axialen Toleranz bei der Erzeugung der Vorspannung oder bei einer Winkeltoleranz oder -abweichung zwischen der Symmetrieachse der Sitztopologie der Aufnahmeeinrichtung und der Symmetrieachse des Ventileinsatzes wirken.

Eine eventuelle laterale Toleranz zwischen den beiden Achsen kann dadurch ausgeglichen werden, dass die erste Abdichtungseinrichtung eine Durchmesserüberlappung mit der zweiten Abdichtungseinrichtung aufweist. Auf diese Weise kann eine Dichtwirkung innerhalb des Überlappungsbereiches gewährleistet werden.

Eine weitere Toleranzausgleichfunktion der Anordnung kann darin bestehen, kleine Unebenheiten, Kerben und/oder Rauhigkeiten in den Dichtoberseiten der beiden Abdichtungseinrichtung zu überbrücken, wozu die Vorspannung in Verbindung mit der effektiven Berührfläche so groß gewählt sein kann, dass sich die erste Abdichtungseinrichtung lokal derart verformt, dass Fehlstellen, wie Unebenheiten, Kerben, Rauhigkeiten und dergleichen mit dem elastischen Material aufgefüllt werden und somit ein dichter Verschluss entsteht.

Zwischen der ersten und der zweiten Abdichtungseinrichtung kann eine ausreichende Flächenpressung an der ringförmigen Berühr- bzw. Verpressungszone im Gebrauch gestaltungsbedingt aufgebaut werden.

Im einfachsten Fall kann, wie in den beigefügten Figuren dargestellt, eine ebene oder im Wesentlichen ebene Form der ersten Abdichtungseinrichtung in der Zone der Abdichtung kombiniert mit einer spitz zulaufenden Form der zweiten Abdichtungseinrichtung vorgesehen sein.

Umgekehrt kann das spitze Element einer solchen einebnungsgeeigneten Geometriepaarung auch ein spitzer ringförmiger Dichtsteg an der ersten Abdichtungseinrichtung sein, welcher auf der in diesem Fall flachen oder leicht gewölbten oder kegelförmigen harten zweiten Abdichtungseinrichtung unter Vorspannung aufliegen kann.

Die Zuordnung spitz zu flach kann vorzugsweise zu einer hohen, für die Einebnung benötigten Flächenpressung führen und dabei sämtliche vorstehend genannten Toleranzausgleichsarten gewährleisten.

Die Dicht- und Toleranzausgleichsfunktionen können nicht nur bei einer flachen Ausgestaltung der Abdichtungseinrichtungen zustande kommen. Sie können ebenso erzielbar sein, wenn die Abdichtungseinrichtungen bewusst und/oder durch axiale Vorspannung zu einer schüsseligen oder kegeligen Form verformt sind.

Wenigstens eine der beteiligten Abdichtungseinrichtungen kann daher in der Form eines spitzen Umlaufsteges ausgeführt sein. Auf diese Weise kann sich auch eine Toleranz oder Unempfindlichkeit gegen die unterschiedlich kegelige und/oder schüsselige Form im Zustand der Verpressung bzw. Dichtung ergeben. Dies kann auch als halbaxiale Dichtweise statt als rein axiale Dichtweise bezeichnet werden.

Dabei können die Vorteile des lateralen Toleranzausgleichs im Gegensatz zu einer rein radialen Dichtweise im vollen Umfang erhalten bleiben.

Die erste Abdichtungseinrichtung wird mittels Kraftübertragung und/oder Wegeinprägung auf den Ventileinsatz mit der zweiten Abdichtungseinrichtung verpressbar oder im Gebrauch verpresst.

Der Begriff "Verpressen", wie er hierin verwendet wird, bezeichnet ein kraft- und/oder formschlüssiges körperliches Verbinden der ersten Abdichtungseinrichtung des Ventileinsatzes und der zweiten Abdichtungseinrichtung der Aufnahmeeinrichtung.

Die übertragene Kraft kann eine von außen zugeführte bzw. aufgebrachte Kraft sein. Die Wegeinprägung kann eine von außen zugeführte bzw. aufgebrachte Wegänderung bzw. Verschiebung sein.

In einer weiter bevorzugten Ausführungsform ist die Kraft auf den Ventileinsatz eine Verpresskraft, die durch den Einbau der Ventilvorrichtung in eine Behandlungsvorrichtung einleitbar ist.

In einer weiter bevorzugten Ausführungsform ist die Wegeinprägung auf den Ventileinsatz eine Verschiebung, die durch den Einbau der Ventilvorrichtung in eine Behandlungsvorrichtung einleitbar ist.

Die Verpresskraft und/oder der eingeprägte Verpressweg können zum Beispiel durch Verpressen der externen Funktionseinrichtung mit der Behandlungsvorrichtung eingeleitet werden. Geeignete Ausführungsformen und Verfahren zum Verpressen einer externen Funktionseinrichtung mit einer Behandlungsvorrichtung sind beispielsweise in den Anmeldungen 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden"* und 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren"* beschrieben, welche von der vorliegenden Anmelderin jeweils am 10. März 2009 beim DPMA eingereicht wurden.

In einer weiter bevorzugten Ausführungsform sind die Verpresskraft und/oder der Verpressweg mittels geeigneter Einrichtungen durch ein Übertragungselement der Behandlungsvorrichtung übertragbar.

Ein solches "Übertragungselement" kann Funktionen der Verpressung und/oder (statischen) Abdichtung des Fluidsystems und/oder Funktion der Einleitung von Kräften und Stellbewegungen ausüben.

Das Übertragungselement kann beispielsweise eine Gummimatte sein. Es kann ein unmittelbarer Koppelpartner der Behandlungsvorrichtung zur externen Funktionseinrichtung sein.

Das Übertragungselement kann durch Anlegen von Steuerfluiden oder durch Ankoppeln von Stößeln schüsselig verformt und ausgelenkt werden.

Die Verformung oder Auslenkung des Übertragungselements kann auf einen Stirnringbereich des Ventileinsatzes übertragen werden.

Das Übertragungselement kann einen Bewegungsüberträger, zum Beispiel einen axialen Bewegungsüberträger, aufweisen. Ein axialer Bewegungsüberträger kann beispielsweise zentral oberhalb eines Ventileinsatzes angeordnet sein und eine auf das Übertragungselement aufgebrachte Kraft und/oder eine Verschiebung auf die Oberseite des Ventileinsatzes übertragen.

Das Übertragungselement kann über eine Ankopplungsfläche aus elastomerem Material verfügen, um Abdichtungs- und/oder Toleranzausgleichsfunktionen zwischen der Aufnahmeeinrichtung und/oder der externen Funktionseinrichtung und der Behandlungsvorrichtung übernehmen zu können. Die Gegenkraft zur Verpressungskraft kann über ein Gegenstück der Behandlungsvorrichtung aufgenommen werden, welches die Stirnfläche der Aufnahmeeinrichtung abstützt.

Es kann auch möglich sein, einen Stirnflächenbereich des Ventileinsatzes vorzusehen, die Funktionen des Übertragungselements der Behandlungsvorrichtung auszuüben. Beispielsweise kann ein solcher Stirnflächenbereich direkt mit einem Stößel (beweglich oder fest) in Kontakt stehen. Der Stirnflächenbereich kann die gewünschten Kräfte und Stellwege in die fluidfunktionellen Bereiche des Ventileinsatzes weiterleiten.

Geeignete Mechanismen sind beispielsweise in den o. g. Anmeldungen der vorliegenden Anmelderin beschrieben, auf deren diesbezügliche Offenbarung hiermit vollumfänglich Bezug genommen wird.

Der Ventileinsatz weist wenigstens eine Führungseinrichtung zum Einführen des Ventileinsatzes in die Aufnahmeeinrichtung auf.

Eine solche "Führungseinrichtung" kann eine Führungsrippe, eine Führungsschiene, ein Zug- und/oder Schiebemechanismus und dergleichen sein.

Der Ventileinsatz weist wenigstens einen Biegeringbereich auf. Der Biegeringbereich ist elastisch.

Der Biegeringbereich kann - wie die Führungseinrichtung - die axiale Beweglichkeit des Ventileinsatzes relativ zur Aufnahmeeinrichtung bei gleichzeitiger Vermittlung der geometrischen Zentrierung zwischen den beiden Symmetrieachsen gewährleisten.

Da es sich aber ähnlich wie bei der Verformung der ersten Abdichtungseinrichtung um eine elastische Verformung aufgrund einer dazu erforderlichen axialen Kraft und/oder Verschiebung handeln kann, kann sich für die Ventilvorrichtung eine Vorzugsstellung ergeben, welche selbsttätig eingenommen werden kann, wenn die von der Behandlungsvorrichtung eingeleiteten äußeren Weg- und Krafteinleitungen wegfallen. Diese Vorzugsstellung kann für die Hauptfunktionen der Ventilvorrichtung von entscheidender Bedeutung sein.

Der Biegeringbereich kann Toleranzaufnahme- oder -ausgleichsfunktionen ausüben. Es kann möglich sein, das Ventilelement systematisch axial tiefer in die Aufnahmeeinrichtung einzubauen. So können in vorteilhafter Weise Einbautiefentoleranzen ausgeblendet werden, da der Ventileinsatz selbstständig in die Vorzugsstellung zurückkehren kann.

Der Biegeringbereich kann einen Stirnringbereich aufweisen. Der Stirnringbereich des Ventileinsatzes kann einen äußeren Stirnringbereich, einen inneren Stirnringbereich, einen Stirnanschlag und einen Verformungsraum, beispielsweise einen ringförmigen Verformungsraum, aufweisen.

Der äußere Stirnringbereich kann eine korrekte Soll-Lage des Ventileinsatzes in der Aufnahmeeinrichtung vermitteln.

Um eine großzügige Durchmessertoleranz und eine großzügige laterale Einbautoleranz zu gewährleisten, kann der äußere Stirnringbereich am Umfang vorzugsweise kegelig ausgeführt sein. Er kann über eine ausreichende radiale Kompressibilität verfügen.

In vorteilhafter Weise können so eine unkritische Einbausituation und daneben in der Montage-Endposition eine hochgenaue spielfreie radiale und axiale Zentrierung erreicht werden.

In Verbindung mit dem Stirnanschlag kann der innere Stirnringbereich eine (wichtige) Funktion des Toleranzausgleichs zwischen der Behandlungsvorrichtung und der externen Funktionseinrichtung ausfüllen. Die relativ geringere Nachgiebigkeit des Materialbereichs zwischen dem Stirnanschlag und der ersten Abdichtungseinrichtung kann in Verbindung mit der relativ hohen axialen Nachgiebigkeit des inneren Stirnringbereichs dafür sorgen, dass die axiale Vorspannung zwischen der Behandlungsvorrichtung und der externen Funktionseinrichtung so gewählt werden kann, dass der Stirnanschlag an einer unteren Stirnfläche der Sitzbuchse der Aufnahmeeinrichtung arretiert wird, während der innere Stirnringbereich durch Verformung die axialen Einbautoleranzen aufnehmen kann. Auf diese Weise kann die korrekte Relativlage der hauptfunktionsrelevanten Bereiche des Ventileinsatzes zum Dichtsitzbereich der Aufnahmeeinrichtung gewährleistet sein.

Eine axiale Materialstrecke oder ein Materialabschnitt zwischen dem äußeren und dem inneren Stirnringbereich kann ausreichend elastisch sein, um unterschiedliche Einbaumaßtoleranzen und Verpressungskräfte zwischen der Behandlungsvorrichtung und der externen Funktionseinrichtung aufzunehmen. Dies kann in vorteilhafter Weise ohne Einfluss auf das hauptfunktionsrelevante Geschehen des eigentlichen Ventileinsatzbereichs erfolgen.

Der äußere Biegeringbereich des Ventileinsatzes kann zusammen mit einem geeigneten Biegeringbereich des Ventileinsatzes bei geeigneter Gestaltung des statischen Dichtsitzes der Aufnahmeeinrichtung (Durchmesserwahl und eventuelle zusätzliche Ausstattung mit statischen umlaufenden Dichtstegen) wahlweise auch radiale und/oder axiale Dichtfunktionen übernehmen. Dies ist besonders vorteilhaft falls beispielsweise keine weitere Abdeckungseinrichtung zwischen der Ventilvorrichtung und der Behandlungsvorrichtung, wie beispielsweise ein Folienelement, vorgesehen ist.

Der ringförmige Verformungsraum, beispielsweise ein ringförmiger Freiraum, kann sich zwischen der ersten Abdichtungseinrichtung, zum Beispiel einer umlaufenden Dichtlippe, und einem Schaft des Ventileinsatzes befinden.

Durch den ringförmigen Verformungsraum kann ein Fluid strömen, wenn die erste Abdichtungseinrichtung auf keiner Dichtfläche aufliegt.

Der ringförmige Verformungsraum kann in vorteilhafter Weise eine Einsparung von axialem Einbauraum für den Ventileinsatz erlauben. Darüber hinaus kann der ringförmige Verformungsraum zusätzliche Durchtrittsbereiche für die Fluide bieten und eine druckverlustarme Verteilung der Fluide vom Strömungskanal auf den gesamten Ringbereich des Dichtsitzes ermöglichen. Somit kann der gesamte potentielle Öffnungsquerschnitt der Ventilvorrichtung genutzt werden.

Der Ventileinsatz kann ferner einen Ventilsitz, beispielsweise einen statischen Ventilsitz, und einen Sitzbereich, bevorzugt einen elastischen Sitzbereich, aufweisen.

Der Ventileinsatz kann in einem zylindrischen oder im Wesentlichen zylindrischen Innenraum, der in Form einer Ausnehmung integraler Bestandteil des Spritzguss-Hartteils einer externen Funktionseinrichtung sein kann, angeordnet sein.

Der Ventileinsatz kann zum Beispiel in einer zylindrischen oder im Wesentlichen zylindrischen Sitzanordnung der Aufnahmeeinrichtung aus einer unteren Sitzbuchse und einer oberen Sitzbuchse gehalten werden. Eine solche geometrische Konstellation kann insbesondere vielfältige Anbindungsmöglichkeiten für die Strömungskanäle bieten.

Eine Führungseinrichtung, beispielsweise in Gestalt einer Mehrzahl von Führungsrippen, kann ein leichtes Übermaß relativ zum Durchmesser der unteren Sitzpartie der Aufnahmeeinrichtung und/oder eine sternförmige Formgebung und/oder eine ballige Formgebung aufweisen.

Die sternförmige Formgebung kann eine korrekte Mittenzentrierung des Ventileinsatzes gewährleisten. Sie kann ferner den freien Zutritt der Fluide zum Dichtsitzbereich bei beliebigen Einbau-Drehlagen des Ventileinsatzes gewährleisten.

Die Ventilvorrichtung kann also frei von Drehpositionier-Anforderungen axial in die Aufnahmeeinrichtung eingebaut werden.

Eine dünne Wandstärke der Führungsrippen kann den Ventileinsatz relativ nachgiebig gegen radiale Kompression machen. Auf diese Weise kann eine große Durchmessertoleranz zur unteren Sitzbuchse bewerkstelligt werden.

Es kann eine, vorzugsweise gleichmäßige, geringe Klemm- oder Verpresskraft erzielt werden, welche in vorteilhafter Weise dafür sorgen kann, dass der Ventileinsatz im Rahmen der Herstellung der externen Funktionseinrichtung nicht aus seinem Sitzbereich herausfallen kann.

Andererseits kann die Klemmkraft so klein sein, dass es beim späteren Gebrauch der Ventilvorrichtung nicht zu einer Verfälschung der Axialkraftverhältnisse bzw. zu einer Behinderung der gewollten Verschiebebewegungen kommen kann.

Vorteilhafterweise kann die Klemmkraft bei einer Sterilisationsbehandlung und während der Lagerungszeit der Ventilvorrichtung bzw. der externen Funktionseinrichtung bis zu ihrem Einsatz noch um ein weiteres Maß abnehmen.

Eine ballige Formgebung der Führungsrippen kann wenigstens zwei weitere Funktionen erfüllen. Bei der Montage kann die ballige, spitz zulaufende Silhouette eine großzügige Einführschräge bilden, so dass sie mittels Hand und/oder Maschineneinbauvorgänge auch bei großen lateralen Toleranzen in vorteilhafter Weise sicher eingebaut werden kann. Zum zweiten kann die Ballenförmigkeit eine kardanische Winkelbeweglichkeit zwischen den Symmetrieachsen der Aufnahmeeinrichtung und des Ventileinsatzes gewährleisten.

In vorteilhafter Weise können somit Materialspannungen und/oder ungewollte Formänderungen des elastischen Dichtringbereiches bei gegebenen Toleranzen in den Formgebungen von Aufnahmeeinrichtung und Ventileinsatz verhindert werden, welche die Dicht- oder Öffnungsfähigkeiten der Ventilvorrichtung beeinträchtigen könnten.

In einer weiter bevorzugten Ausführungsform ist die Ventilvorrichtung an wenigstens einer Oberseite mit einer Abdeckungseinrichtung versehen.

Die Abdeckungseinrichtung kann auf die Oberseite des Gehäuseelements der Aufnahmeeinrichtung aufgeklebt, aufgeschweißt, aufgelötet, mit dieser vernietet und dergleichen werden.

Die Abdeckungseinrichtung kann ein Folienelement sein. Sie kann eine nachgiebige Folie sein.

Folien sind kostengünstig und mechanisch nachgiebig. Mit ihnen können auf preiswerte Art Ventilstellen in einem Fluidsystem mit einer oder mehreren Einwirkungsstellen für Ventilstellungsbewegungen durch die Behandlungsvorrichtung ausgestaltet werden.

Anstelle der Folien können zur Einleitung der erforderlichen Bewegungen eingelegte, im Zweikomponentenverfahren eingespritzte oder auf andere Weise eingefügte elastomere Ventilschaltmatten verwendet werden. Ebenso ist eine Kombination aus Hartteilen, Folien und/oder Elastomerkörpern von der vorliegenden Erfindung umfasst.

Die Abdeckungseinrichtung kann Fluidkanäle darstellen, den Ventileinsatz in einer axialen Dimension oder Richtung halten, das Fluidsystem in Verbindung mit der Durchlässigkeit für axiale Steuerbewegungen auf den Ventileinsatz nach außen fluiddicht abschließen und Verpresskräfte zur Steuerung oder Regelung des Ventileinsatzes und zur Abdichtung der Aufnahmeeinrichtung bzw. der externen Funktionseinrichtung übertragen.

Die Abdeckungseinrichtung kann das Fluidkanalsystem der Aufnahmeeinrichtung durch Verpressung mit dem statischem Dichtsteg der Aufnahmeeinrichtung abschließen bzw. abdichten. Dabei - oder zu diesem Zweck - kann ein statischer Dichtsteg, beispielsweise in Form einer linienförmigen Delle gebildet werden.

Das Folienelement kann eine ausreichende, elastische Nachgiebigkeit aufweisen, zum Beispiel durch elastische Materialeigenschaften und/oder durch geeignete Balg-Prägungen. Da aufgrund der Balg-Prägungen lokal überwiegend nur Biegespannungen auftreten, kann mit den Balg-Prägungen, beispielsweise in Ausgestaltungen des Folienelements als Rollmembran oder Rollbalg, in vorteilhafter Weise die bei flach gespannten, auf Durchbiegung beanspruchten Membranen des Standes der Technik oftmals in der Membranebene auftretenden Zugspannungen verhindert oder zumindest merklich verringert werden.

Aufgrund der elastischen Eigenschaften der Abdeckungseinrichtung kann - parallel oder alternativ zur statischen Dichtfunktion - auch die Funktion der mechanischen Bewegungs- und/oder Kraftübertragung von der Behandlungsvorrichtung auf den Ventileinsatz gewährleistet sein.

In einer weiter bevorzugten Ausführungsform ist der Ventileinsatz daher mittels einer von einer Behandlungsvorrichtung übertragenen Kraft bzw. Verschiebung durch die Abdeckungseinrichtung hindurch schaltbar.

Alternativ kann die Abdeckungseinrichtung - je nach Anwendungsfall - auch ganz wegfallen. Beispielsweise kann der Ventileinsatz durch Verschweißung oder durch Verpressung gegen die Aufnahmeeinrichtung abgedichtet sein und dadurch die Funktionen der Abdichtung und der Bewegungsübertragung zur Behandlungsvorrichtung mit übernehmen.

Dies kann vorteilhafter Weise in besonders einfacher Weise erfolgen, wenn beide Strömungskanäle als geschlossene Kanäle ausgeführt sind. Ebenso kann es Anwendungsfälle geben, etwa im Falle reduzierter Ansprüche an die Fremdstoff- und/oder Keimfreiheit des Fluidsystems, in denen das zur Behandlungsvorrichtung gehörende Übertragungselement die Funktionen der Abdeckungseinrichtung mit übernimmt.

Die erfindungsgemäße externe Funktionsvorrichtung weist einen Ventileinsatz auf, welcher in einer Ventilvorrichtung, wie sie vorstehend beschrieben ist, eingesetzt werden kann.

Ein solcher Ventileinsatz kann das Kernelement der offenbarten Ventilvorrichtung bilden. Seine Hauptfunktion kann darin bestehen, einen Strömungskanal in Abhängigkeit vom Funktionsmodus bzw. Ventilzustand durch eine axiale Hubbewegung des gesamten Ventileinsatzes im Falle der Ventilzustände (z. B. Sterilisation, Auf, Zu) oder durch eine Verformung wie einer Verbiegung unter dem Einfluss des Fluids (z. B. Rückschlag, Steuer/Regel) zu öffnen oder zu verschließen.

Die externe Funktionseinrichtung kann beispielsweise zur extrakorporalen Blutbehandlung eingesetzt werden. Bei der externen Funktionseinrichtung kann es sich um eine Disposable-Kassette handeln, wie sie in den von der vorliegenden Anmelderin mit dem Titel *"Externe Funktionseinrichtung, Blutbehandlungsvorrichtung zum Aufnehmen einer erfindungsgemäßen externen Funktionseinrichtung, sowie Verfahren"* beim DPMA eingereichten Patentanmeldungen wie oben aufgeführt beschrieben wurde.

Da sich alle im Zusammenhang mit der offenbarten Ventilvorrichtung diskutierten oder im Folgenden genannten Vorteile ungeschmälert auf den Ventileinsatz und/oder die externe Funktionseinrichtung übertragen lassen, wird zur Vermeidung von Wiederholungen auf die entsprechenden Ausführungen verwiesen.

Offenbart sind ferner verschiedene Verfahren vorgeschlagen, bei denen die offenbarte Ventilvorrichtung vorteilhaft einsetzbar ist.

Ein offenbartes Verfahren betrifft ein Verfahren zum Steuern oder Regeln eines Fluiddurchgangs, welches das Verwenden der offenbarten Ventilvorrichtung umfasst.

Unter dem Begriff "Fluiddurchgang" kann ein Fluiddurchlass, ein Fluidfluss, eine Fluidströmung und dergleichen verstanden werden.

Das "Steuern oder Regeln" eines Fluiddurchgangs schließt, ohne darauf beschränkt zu sein, das Steuern oder Regeln einer Geschwindigkeit, eines Drucks, einer Durchflussmenge, einer Fließrichtung und dergleichen der die offenbarte Ventilvorrichtung durchströmenden Fluide ein.

Das Verfahren kann ferner das Aufbringen einer definierten Kraft und/oder einer definierten Verschiebung auf eine Oberseite des Ventileinsatzes zum Schalten des Ventileinsatzes umfassen.

Die gezielte oder "definierte" Kraft und/oder die gezielte oder "definierte" Verschiebung kann - wie vorstehend ausgeführt - in Abhängigkeit von dem erwünschten oder erforderlichen Ventilzustand des Ventileinsatzes aufgebracht werden.

Die offenbarte besonders bevorzugte Betätigung der Ventilvorrichtung ist eine wegorientierte. Eine kraftorientierte Betätigung ist jedoch gleichermaßen von der vorliegenden Erfindung umfasst.

In einer weiter bevorzugten Ausführungsform werden die Kraft und/oder die Verschiebung mittels eines Übertragungselements auf die Oberseite des Ventileinsatzes aufgebracht.

In einer weiter bevorzugten Ausführungsform werden die Kraft und/oder die Verschiebung durch eine Abdeckungseinrichtung, beispielsweise ein Folienelement, hindurch auf den Ventileinsatz aufgebracht.

In einer weiter bevorzugten Ausführungsform ist die Kraft eine Verpresskraft, welche durch Einbau einer offenbarten Ventilvorrichtung oder einer externen Funktionseinrichtung, welche eine offenbarte Ventilvorrichtung aufweist, in eine Behandlungsvorrichtung eingeleitet wird.

In einer weiter bevorzugten Ausführungsform wird die Verschiebung durch Einbau einer offenbarten Ventilvorrichtung oder einer externen Funktionseinrichtung, welche eine offenbarte Ventilvorrichtung aufweist, in die Behandlungsvorrichtung eingeleitet.

Geeignete Behandlungsvorrichtungen können medizintechnische Behandlungsvorrichtungen, wie beispielsweise eine Blutbehandlungsvorrichtung, etwa eine Dialysevorrichtung, eine Hämodialysevorrichtung, eine Vorrichtung zur Hämofiltration oder zur Hämodiafiltration und dergleichen, sein. Sie können ferner Vorrichtungen in der Labortechnik, wie Analysevorrichtungen, wie Chromatographievorrichtungen, Waagen, und dergleichen, Vorrichtungen in der Nahrungs- und/oder Arzneimittelherstellung oder dergleichen sein.

Alternativ können die Kräfte auf die Ventilvorrichtung auch durch die Strömung von Fluiden, welche die Ventilvorrichtung durchströmen, hervorgerufene Kräfte sein.

Da mit dem Verfahren alle mit der offenbarten Ventilvorrichtung erzielbaren Vorteile ungeschmälert erreichbar sind, wird zur Vermeidung von Wiederholungen auf die entsprechende Ausführung derselben verwiesen.

Offenbart ist ferner die Herstellung einer Ventilvorrichtung mit einem Herstellungsverfahren. Das Verfahren umfasst das Positionieren eines Ventileinsatzes über einer Aufnahmeeinrichtung und das Einsetzen des Ventileinsatzes in die Aufnahmeeinrichtung unter Zuhilfenahme einer Führungseinrichtung.

Das Positionieren des Ventileinsatzes kann maschinell oder maschinengesteuert, beispielsweise mit Hilfe eines Roboters, erfolgen. Geeignete Steuer- oder Regelvorrichtungen können vorgesehen sein.

Das Einsetzen des Ventileinsatzes in die Aufnahmeeinrichtung kann ausgeführt werden, indem der Ventileinsatz während des Einsetzens an seiner Oberseite von einem Saugnapf an einer Einsetzvorrichtung gehalten wird.

Selbstverständlich kann jede weitere geeignete Vorrichtung zum Positionieren und/oder Einsetzen des Ventileinsatzes verwendet werden, und die Erfindung ist nicht auf die hierin genannten Beispiele beschränkt.

Ferner kann eine externe Funktionseinrichtung mit einem Herstellungsverfahren hergestellt werden, welches das Herstellen eines Gehäuseelements aus wenigstens einem Thermoplast oder Duroplasten, das Ausbilden einer Aufnahmeeinrichtung im Gehäuseelement, das Einsetzen eines Ventileinsatzes und das Aufbringen einer Abdeckungseinrichtung auf wenigstens einen Abschnitt einer Oberseite des Gehäuseelements umfasst.

Die Herstellung des Gehäuseelements kann beispielsweise mittels Spritzgusses erfolgen.

Das Ausgestalten der Aufnahmeeinrichtung kann während der Herstellung des Gehäuseelements, beispielsweise mittels Formguss- oder Spritzgusstechniken erfolgen. Es kann jedoch auch nach Herstellung des Gehäuseteils durch Herausarbeiten der Aufnahmeeinrichtung, wie beispielsweise durch Fräsen, Schleifen und dergleichen, erfolgen.

In der Erfindung ist ebenfalls offenbart, eine zum Aufnehmen der Aufnahmeeinrichtung geeignete Aussparung im Gehäuseelement vorzusehen oder vorzubilden und eine separat hergestellte Aufnahmeeinrichtung in geeigneter Weise in die Aussparung einzusetzen oder einzufügen. Eine separat gefertigte Aufnahmeeinrichtung kann mit dem Gehäuseelement bzw. dem Hartteil, der externen Funktionseinrichtung kraft- und/oder form- und/oder stoffschlüssig verbunden werden, beispielsweise verklebt, verschweißt, verhakt, verzahnt werden und dergleichen.

Der Ventileinsatz kann von der offenen Seite der externen Funktionseinrichtung her in die Aufnahmeeinrichtung eingesetzt werden.

Anschließend kann ein Ankoppelabschnitt, z. B. in Gestalt einer Ankoppelebene, der externen Funktionseinrichtung mit der Abdeckungseinrichtung, zum Beispiel einer Folie, verschweißt werden.

Der Ventileinsatz kann im Inneren der externen Funktionseinrichtung unter der Folie derart liegen, dass er durch einen Aktor, der auf die Abdeckungseinrichtung drückt, bei verschiedenen definierten Wegstufen und/oder Kraftstufen des Aktors unterschiedlich und insbesondere vorbestimmt tief in die Aufnahmeeinrichtung hineindrückbar ist. Es können sich somit verschiedene, definierte, elastische Verformungszustände des Ventileinsatzes ergeben. Diese können verschiedene definierte Durchflussmengen der die Ventilvorrichtung durchströmenden Fluide bewirken.

Ein weiteres offenbartes Verfahren betrifft das Vorbereiten der externen Funktionseinrichtung zur sterilen Verwendung mit einem Verfahren, welches das Herstellen einer externen Funktionseinrichtung, wie es beispielsweise vorstehend beschrieben ist, und das Sterilisieren der mit dem Herstellungsverfahren erhaltenen externen Funktionseinrichtung umfasst.

Die externe Funktionseinrichtung weist eine offenbarte Ventilvorrichtung auf, deren Ventileinsatz sich im - insbesondere eigens dafür vorgesehenen - Sterilisationszustand befindet. Der Sterilisationszustand kann eine Durchfluss-Stellung sein, in welcher die erste Abdichtungseinrichtung nicht auf der zweiten Abdichtungseinrichtung aufliegt. So kann in vorteilhafter Weise ein bidirektionaler Durchfluss der die offenbarte Ventilvorrichtung durchströmenden Fluide möglich sein. Eine solche Stellung kann zum Sterilisieren der externen Funktionseinrichtung besonders geeignet sein.

Das Sterilisieren der externen Funktionseinrichtung kann mittels eines Dampfverfahrens, eines Dampf-Vakuumverfahrens eines Ethylenoxidverfahrens oder dergleichen erfolgen. Es ist ausdrücklich nicht auf diese beispielhaft genannten Sterilisationsverfahren beschränkt. Ein geeignetes Sterilisationsverfahren kann je nach Verwendungszweck der externen Funktionseinrichtung und entsprechend den Anforderungen an die Sterilität der externen Funktionseinrichtung im Einzelfall bestimmt werden.

Nach dem Sterilisieren kann die Ventilvorrichtung in einer weiteren bevorzugten Ausführungsform des offenbarten Verfahrens in eine Verriegelungsstellung überführt werden.

Die Verriegelungsstellung kann eine Stellung, in welcher die Ventilvorrichtung lediglich vor dem Eindringen von Fremdsubstanzen geschützt ist, jedoch keine äußere Kraft und/oder Vorspannung auf den Ventileinsatz der Ventilvorrichtung wirkt. Der Ventileinsatz kann in die Aufnahmeeinrichtung eingeklemmt sein, welche die Selbsteinstellung des initialen Spaltmaßes in vorteilhafter Weise nicht behindern.

In dieser Stellung kann die externe Funktionseinrichtung gelagert werden.

Die Verriegelungsstellung ist bevorzugt identisch mit der Stellung, die der Ventileinsatz im Einsatz bei der Fertigung selbsttätig einnimmt. Diese Stellung kann vorteilhaft das Herausfallen eines in eine Kassette eingebauten Ventileinsatzes beim Wenden der Kassette sowohl bei der Fertigung als auch bei der Sterilisation, Lagerung, beim Transport und/oder während einer Behandlung verhindern.

Die Verriegelungsstellung ist bevorzugt identisch mit der Sterilisationsstellung (Dampfsterilisationsstellung) und/oder mit der Auslieferungsstellung, bis ausschließlich des Schließens eines Abdeckungselements bzw. einer Verpressungstür der Behandlungsvorrichtung.

Die vorliegende Erfindung schlägt ferner auch eine externe Funktionseinrichtung zur sterilen Verwendung vor, welche mit dem vorstehend beschriebenen Verfahren sterilisiert wurde.

Eine solche externe Funktionseinrichtung kann beispielsweise in einem medizintechnischen und/oder labortechnischen Verfahren oder zur Herstellung von Nahrungsmitteln und/oder Arzneimitteln eingesetzt werden. Beispiele hierfür schließen eine Verwendung externer Funktionseinrichtungen mit einer Behandlungsvorrichtung ein, wie sie vorstehend unter Bezugnahme auf die Behandlungsvorrichtungen selbst beschrieben wurden.

Die externe Funktionseinrichtung kann eine Blutbehandlungskassette mit blutführenden Kanälen für die extrakorporale Blutbehandlung sein, wobei die Ventilvorrichtung (100) vorgesehen und ausgelegt sein kann, um über sie Substituat und/oder eines oder mehrere Medikamente in die blutführenden Kanäle einzubringen.

Die vorliegende Erfindung schlägt des Weiteren auch eine Behandlungsvorrichtung vor, welche wenigstens eine offenbarte Ventilvorrichtung und/oder eine erfindungsgemäße externe Funktionseinrichtung aufweist. Eine solche Behandlungsvorrichtung kann beispielsweise als Blutbehandlungsvorrichtung ausgestaltet sein.

Da die offenbarten Ventilvorrichtung ein wesentliches Element aller weiteren erfindungsgemäßen Ausführungen und Ausgestaltungen darstellt, ergeben sich für alle weiteren Aspekte und Verwendungs- und Einsatzmöglichkeiten derselben ungeschmälert alle Vorteile, welche mit der Ventilvorrichtung erzielbar sind.

Fluidführende Einrichtungen wie externe Funktionseinrichtungen müssen i. a. R. so ausgeliefert werden, dass alle durch die Fluide im späteren Gebrauch benetzbaren Räume und Oberflächen anfänglich schadstofffrei und steril sind und während der Benutzung nach außen hin hermetisch dicht bleiben. Dies kann gewöhnlich durch gegeneinander mit Elastomerdichtungen verpresste oder miteinander verschweißte Gehäusekomponenten ermöglicht werden. Auch Folien können die Aufgabe von Gehäusekomponenten übernehmen und durch Verpressung oder Verschweißung fluiddicht gegen andere Gehäusekomponenten gemacht werden.

In vorteilhafter Weise kann der elastische Ventileinsatz der offenbarten Ventilvorrichtung durch geeignete geometrische Gestaltung verschiedene Ventilfunktionen (z. B. "terilisation", "Offen", "Geschlossen", "Regeln", "Rückschlag") in nur einem Element darstellen.

Gerade in der Stellung einer Rückschlag-Ventilfunktion kann der elastische Ventileinsatz aufgrund seiner geometrischen Gestaltung beispielsweise gegenüber den bekannten vorgespannt zu montierenden Rückschlagventilen einen vorteilhaft geringeren Montageaufwand erfordern.

Durch das Verwenden der offenbarten Ventilvorrichtung lassen sich Einbauräume und Ansteuereinrichtungen für die Ventilvorrichtung platz- und kostengünstig schaffen, welche die oben genannten Funktionen auf neue, sichere, einfache und/oder kostensparende Art erfüllen können.

Der elastische Ventileinsatz kann in vorteilhafter Weise erstmals in einem Bauteil und mit nur einem einzigen Bauraumbedarf die Funktionen mehrerer Ventilarten vereinigen. Zur Nutzung dieser Ventilarten muss keine Ventilvorrichtung der externen Funktionseinrichtung körperlich verändert oder ausgetauscht oder eine Mehrzahl von Ventilen vorgesehen werden. Es genügt vielmehr, in vorteilhafter Weise nur die Auslegung oder die Steuerung oder Regelung der Behandlungsvorrichtung zum Betreiben des Ventils und zum Umschalten zwischen verschiedenen Ventilzuständen entsprechend zu variieren.

Aus dem Stand der Technik bekannte passiv arbeitende Rückschlagventilelemente sind im Allgemeinen fest und vorgespannt in die Einmalteil-Gehäusekomponenten eingebaut. Sie sind beispielsweise unter Vorspannung in Haltebohrungen von harten Gehäusekomponenten eingeklipst oder zwischen zwei harte Gehäusekomponenten eingeklemmt (zum Beispiel die pilzförmigen "umbrella valves", Firma Minivalve International, Niederlande, http://www.minivalve.com).

Bei Sterilisationsbehandlungen unter erhöhter Temperatur und generell durch Lagerung können diese Elastomerbauteile einen Teil ihrer Vorspannung verlieren und so die gewünschte Funktion einbüßen. Weiterhin können diese Elemente die Fluiddurchströmung in beide Richtungen bis zu einem bestimmten Ansprech-Differenzdruck in die eine Richtung und gegen den maximalen Auslegungs-Differenzdruck des Fluidsystems in die andere Richtung unterbinden. In vielen Fällen kann daher eine Sterilisation mit Sterilisationsverfahren, bei welchen die Durchströmung der Fluidräume erforderlich ist, unmöglich oder nur eingeschränkt möglich sein (zum Beispiel Dampf-Vakuum-Sterilisation oder Ethylenoxidsterilisation). Eine Vakuum-Dampf-Sterilisation von externen Funktionseinrichtungen, insbesondere Einmalteilen aus Folien und Hartteilen, mit durch die Folien verschließbaren halboffenen Fluidkanälen kann häufig zu zerstörungsbehafteten Tiefzieheffekten führen. So können beispielsweise die auf ihre Plastizitätsgrenze erwärmten Folien beim Wechsel von Vakuum auf Überdruck in die halboffenen Fluidkanäle gezogen werden. Dies kann insbesondere dann der Fall sein, wenn die Rückschlagventile so angeordnet werden müssen, dass sie die einzigen Fluidzugänge der Kanäle verschließen, welche auf der Innenseite der Folie angeordnet sind.

Anders als fest eingebaute und vorgespannte Rückschlag-Ventilvorrichtungen aus dem Stand der Technik kann die offenbarte Ventilvorrichtung während eines Sterilisationsverfahrens eine Fluiddurchströmung in vorteilhafter Weise in beide Richtungen zulassen. Dies kann besonders vorteilhaft sein, wenn Sterilisationsverfahren eingesetzt werden, die eine Durchströmung von Fluidräumen erfordern.

Ferner kann es in vorteilhafter Weise möglich sein, auf einfachere und/oder zuverlässigere Weise zu sterilisieren. Dabei können besonders preiswerte elastische Werkstoffe für den Ventileinsatz eingesetzt werden, welche zuvor wegen mangelnder Beständigkeit bei gegebenen Sterilisationsbedingungen infolge ihrer Vorspannung nicht oder nur unter Inkaufnahme von Sterilisationsverlusten verwendbar waren.

In vorteilhafter Weise kann es möglich sein, dass geeignete elastische Werkstoffe nicht mehr besonders gegen Kriechverformung beständige Elastomere wie technisch aufwendig herzustellendes und teures Silikon umfassen müssen.

Fest vorgespannte Rückschlagventile des Standes der Technik können im Behandlungseinsatz die Fluidströmung in die Gegenrichtung verhindern, wodurch häufig bestimmte Behandlungsprogramme und initiale Integritätstestprogramme des Einmalteilsystems nicht durchführbar sind.

Mit der offenbarten Ventilvorrichtung kann der Fluiddurchgang durch die Maschinensteuerung in vorteilhafter Weise beliebig geöffnet und verschlossen werden, ohne hierfür ein parallel oder in Reihe geschaltetes weiteres Fluidsteuerungselement zusätzlich vorsehen zu müssen. Die offenbarte Ventilvorrichtung kann demnach in vorteilhafter Weise dazu beitragen, Bauräume- und/oder Toträume zu verringern.

Die offenbarten Ventilvorrichtung kann in vorteilhafter Weise erstmals eine strömungsoffene und mechanisch spannungsfreie Herstellung, Behandlung und Lagerung ermöglichen. Dabei reicht es, wenn die offenbarte Ventilvorrichtung - anders als herkömmliche Ventilvorrichtungen - erst durch ihren Einbau in die Behandlungsvorrichtung in einen vorgespannten Ventilzustand gebracht wird.

Die Fähigkeit der Ventilvorrichtung, neben der Rückschlagventilfunktion auch maschinengesteuert auf und zu gefahren werden zu können, kann in vorteilhafter Weise eine Zunahme der verfahrenstechnischen Möglichkeiten in fluidischen Anordnungen erlauben.

Durch Verwenden der offenbarten Ventilvorrichtung als mehrstufig oder kontinuierlich arbeitendes passiv wirkendes oder aktiv veränderbares Druck- oder Volumenstromregelventil können in vorteilhafter Weise Kosten, konstruktiver Aufwand sowie Bauraum eingespart werden.

Die offenbarte Ventilvorrichtung ist eine Multifunktions-Ventilvorrichtung, welche sich in vorteilhafter Weise sowohl für den direkten Einsatz in mehrfach verwendbaren Behandlungsvorrichtungen als auch in einmalig benutzbaren hermetisch abgeschirmten Disposable-Anordnungen in labortechnischen und medizintechnischen Anwendungsgebieten eignen kann.

Die erfindungsgemäße externe Funktionseinrichtung kann viele verschiedene Funktionen beherbergen. Sie kann kostengünstig zur Aufnahme des Ventileinsatzes ausgestaltet sein. Besondere konstruktive Maßnahmen sind hierzu nicht erforderlich.

Da sich die offenbarte Ventilvorrichtung während ihrer Herstellung und Lagerung in einem Sterilisationsventilzustand, d. h. einem Zustand ohne Vorspannung, befindet, können gerade bei Sterilisationsverfahren nach dem Überdruck-Unterdruck-Wechselverfahren in vorteilhafter Weise kritische Bereiche einer eine solche Ventilvorrichtung aufweisenden externen Funktionseinrichtung effektiv durchströmt werden. Solche kritischen Bereiche könnten bei den üblicherweise vorgespannt und damit geschlossen eingebauten Rückschlagventilen aufgrund ungenügend sterilisierbarer Sackloch-Anordnungen entweder vom Zutritt der Behandlungsfluide ausgeschlossen sein oder nur ineffektiv bespült werden.

Da die offenbarte Ventilvorrichtung bei ihrer Sterilisation und Lagerung ohne Vorspannung vorliegt, können die mechanischen Dimensionen und elastischen Eigenschaften des Ventileinsatzes in vorteilhafter Weise bis zum Zeitpunkt des Einsatzes vollständig und in voller Qualität erhalten bleiben.

Die spannungsfreie Lagerung des Ventileinsatzes kann darüber hinaus in vorteilhafter Weise die Verwendung besonders preiswerter thermoplastischer Elastomere anstelle der üblicherweise erforderlichen herkömmlichen Elastomere (im medizintechnischen Bereich in der Regel Silikongummi) erlauben.

Ferner kann der Einsatz thermoplastischer Elastomere in vorteilhafter Weise neue konstruktive Möglichkeiten erlauben. Eine thermoplastisch-elastomere Mischung für den Ventileinsatz, welche sowohl Elastomere als auch die für die Gehäuseelemente einsetzbaren Thermoplaste umfasst, kann in geeigneter, vorteilhafter und/oder verbesserter Weise mit den Gehäuseelementen verbindbar sein.

So kann es beispielsweise in vorteilhafter Weise möglich sein, Verschweißungen zwischen den elastischen Ventilelementen und den thermoplastischen harten Gehäusekomponenten zwecks Montage und Dichtung des Ventilelements gegen das Gehäuse vorzunehmen.

Durch den Einbau der externen Funktionseinrichtung in die Behandlungsvorrichtung kann nicht nur die korrekte Lage, Abstützung und Fluiddichtigkeit der externen Funktionseinrichtung hergestellt werden. Im Gegensatz zu herkömmlichen Anordnungen mit vorgespannten Rückschlagventilen kann in der offenbarten Ventilvorrichtung in vorteilhafter Weise auf weitere, z. B. parallel geschaltete Fluidkanäle mit zusätzlichen maschinenbetätigten Ventilen, um die Fluidanordnung bidirektional für die Durchströmung zu öffnen, verzichtet werden. Somit können in vorteilhafter Weise nicht nur Einbauräume und Herstellkosten bei der Maschine bzw. Behandlungsvorrichtung und der externen Funktionseinrichtung eingespart, sondern ebenfalls unerwünschte Strömungstoträume vermieden werden.

Die Fähigkeit, Toleranzen beim Einbauen und Ankoppeln der externen Funktionseinrichtung in die Behandlungsvorrichtung zuzulassen, kann in vorteilhafter Weise Funktionsbeeinträchtigungen der einzelnen Bauteile vermeiden. Zudem trägt sie zu günstigen Herstellkosten bei.

Daneben kann in vorteilhafter Weise ein Überprüfen der Ventilfunktionen der offenbarten Ventilvorrichtung über maschinengesteuerte Routinen vor Beginn der eigentlichen Behandlungseinsätze auf einfache und leichte Art und Weise möglich sein. Eine solche Überprüfung kann beispielsweise ein Druckhaltetest sein, welcher einfach und leicht durchführbar ist, indem die offenbarte Ventilvorrichtung geöffnet wird, ein Testfluid in einen kompressiblen Raum entgegen der bei herkömmlichen Rückschlagventilen möglichen Richtung gedrückt wird und die offenbarte Ventilvorrichtung dann in Rückschlagventilposition gebracht wird.

Da der axiale, elastische Sitzbereich des Ventileinsatzes und/oder der elastische Dichtring mit definierter Nachgiebigkeit ausgestaltet werden können, kann es in vorteilhafter Weise möglich sein, einen fest eingestellten oder einen bei Bedarf verstellbaren Druckregler oder Volumenstromregler preisgünstig zu realisieren.

Die geometrische Gestaltungsfreiheit des axialen elastischen Sitzbereichs und/oder des elastischen Dichtrings hinsichtlich deren Steifigkeit kann vorteilhaft erlauben, die Progressivität der Federrate der elastischen Abdichtungseinrichtungen in der Verpress-Situation der Ventilvorrichtung in vorteilhafter Weise in weiten Grenzen einzustellen.

Die Ansteuerung des Ventils kann in einer bevorzugten Ausführungsform voranging wegorientiert erfolgen, wobei die Tatsache, dass mit den vorgegebenen Verformungen natürlich auch Kräfte wie z. B. Rückstellkräfte und Vorspannungen verbunden sind, von geringer oder ohne Bedeutung ist. Letztere sind u.a. deshalb vernachlässigbar, da sie konstruktiv bedingt sehr klein sein können. Es sind vielmehr die definierten Wegeinprägungen, welche erhebliche Vorteile gegenüber der alternativ erfindungsgemäß ebenfalls möglichen Vorgabe von Kräften bieten. So ist die genaue Einhaltung von betragsmäßig kleinen Kräften aufgrund unvermeidbarer Krafttoleranzen und sonstiger Unwägbarkeiten wesentlich aufwändiger und unzuverlässiger als das Einhalten von Wegeeinprägungen und Verschiebungen. Ein großer Vorteil der offenbarten Ventilvorrichtung besteht darin, dass die Wegänderungen auf einfache Weise präzise eingehalten werden können. Dabei können sogar Gesamttoleranzen (z. B. den Ventilkörper betreffend) zuverlässig ausgeglichen werden.

Eine elastische Konstruktionsweise des Ventileinsatzes kann vorteilhaft zu erhöhter Toleranz-Unempfindlichkeit gegenüber Wegeinprägungstoleranzen führen.

In vorteilhafter Weise kann dies insbesondere die Ausführung der Ventilvorrichtung als (durch Einbau) fest eingestelltes Volumenstrom-Konstantregelventil erlauben. Die Ventilvorrichtung kann somit in vorteilhafter Weise als einfache und passiv wirkende Anordnung ausgeführt werden, die abhängig vom Volumenstrom den Durchtrittswiderstand verändern kann, was zu einer Regulierung des Volumenstroms nach dem Prinzip des Proportionalreglers führen kann.

Wo immer in der vorliegenden Erfindung von "Fluiden" die Rede ist, umfasst die vorliegende Erfindung stets dann, wenn das jeweils Ausgeführte für den Fachmann erkennbar auch auf ein einzelnes Fluid oder Mischungen von Fluiden zutreffen kann, alternativ genau ein Fluid oder eine Mischung. Auch der umgekehrte Fall ist von der vorliegenden Erfindung umfasst.

Dabei gilt im Zusammenhang mit der vorliegenden Offenbarung, dass die Ausdrücke "kann aufweisen", "kann sein" und dergleichen synonym zu den hier und auch an anderer Stelle ebenfalls verwendeten Ausdrücken "weist bevorzugt auf, "ist bevorzugt" und dergleichen sind.

Im Folgenden wird die offenbarten Ventilvorrichtung anhand bevorzugter Ausführungsformen derselben unter Bezugnahme auf die Zeichnungen beschrieben. In den Figuren der Zeichnungen bezeichnen gleiche Bezugszeichen gleiche oder identische Elemente. Es gilt:
- Fig. 1: zeigt eine Explosionsdarstellung von Komponenten einer Ventilvorrichtung;
- Fig. 2: zeigt eine schematisch vereinfachte Seitenansicht eines Ventileinsatzes einer Ventilvorrichtung;
- Fig. 3: zeigt schematisch eine seitliche Ansicht einer Ventilvorrichtung im Schnitt in einem Ventilzustand einer Sterilisations- oder Lagerungsstellung;
- Fig. 4: zeigt schematisch eine seitliche Ansicht einer Ventilvorrichtung im Schnitt in einem Ventilzustand einer permanent geöffneten Ventil stellung;
- Fig. 5: zeigt schematisch eine seitliche Ansicht einer Ventilvorrichtung im Schnitt in einem Ventilzustand eines geschlossenen Rückschlagventils;
- Fig. 6: zeigt schematisch eine seitliche Ansicht einer Ventilvorrichtung im Schnitt in einem Ventilzustand eines geöffneten Rückschlagventils;
- Fig. 7: zeigt schematisch eine seitliche Ansicht einer Ventilvorrichtung im Schnitt in einem Ventilzustand als permanent geschlossenes Ventil oder als Regelventil;
- Fig. 8: zeigt eine Seitenansicht einer an ihrer Vorderseite mit einer Abdeckungseinrichtung versehenen erfindungsgemäßen externen Funktionseinrichtung gemäß einer bevorzugten Ausführungsform;
- Fig. 9: zeigt die externe Funktionseinrichtung der Fig. 8 mit aufgeklappter Abdeckungseinrichtung; und
- Fig. 10: zeigt die externe Funktionseinrichtung aus Fig. 9 und Fig. 10 von ihrer Rückseite.

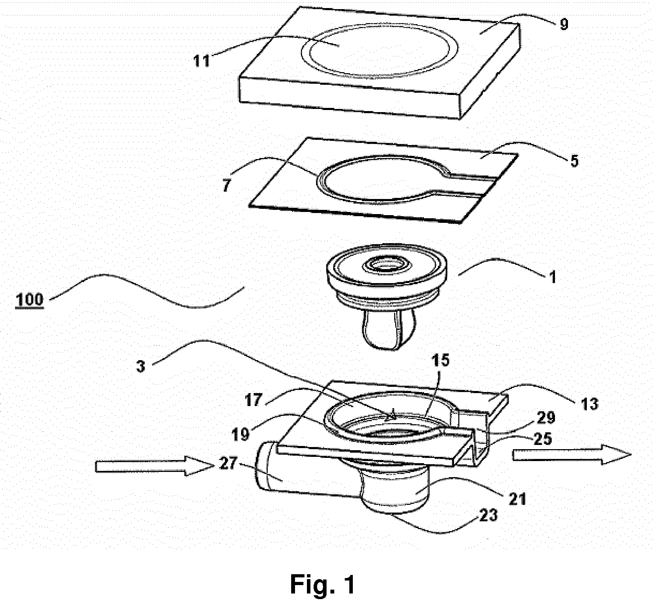

Die Ventilvorrichtung 100 weist, wie in der Explosionsdarstellung der Fig. 1 gezeigt, einen Ventileinsatz 1 auf, welcher in eine Aufnahmeeinrichtung 3 eingesetzt wird. Der Ventileinsatz 1 wird an seiner Oberseite mit einer Abdeckungseinrichtung, beispielsweise einem Folienelement 5, abgedeckt, welches einen statischen Dichtverlauf 7 aufweist.

Der Dichtverlauf 7 kann erfindungsgemäß beispielsweise in Form eines Dichtrings oder eines Dichtrands, einer Delle oder dergleichen ausgestaltet sein.

Über dem Folienelement 5 ist ein Übertragungselement 9 angeordnet, welches einen Bewegungsüberträger 11 zum Übertragen von Bewegungen oder Kräften in axialer Richtung des Ventileinsatzes (in Fig. 1 eine "von oben nach unten"-Richtung) oder umgekehrt aufweist.

Das Übertragungselement 9 kann maschinenseitig angeordnet sein, d. h. Teil einer in Fig. 1 nicht gezeigten Behandlungsvorrichtung, wie beispielsweise einer Behandlungsvorrichtung zum Behandeln von Blut, sein.

Die Aufnahmeeinrichtung 3 ist in einem Gehäuseelement 13 vorgesehen. Das Gehäuseelement 13 kann, wie es beispielsweise in der Fig. 1 gezeigt ist, Teil einer externen Funktionseinrichtung (nicht gezeigt) sein.

Das Gehäuseelement 13 kann aus einem steifen oder relativ steifen Werkstoff, wie beispielsweise einem Thermoplast, gefertigt sein.

Die Aufnahmeeinrichtung 3 weist eine zweite Abdichtungseinrichtung, zum Beispiel in Form eines starren Dichtrings 15 sowie eine obere Sitzbuchse 17, einen statischen Dichtring 19 und eine untere Sitzbuchse 21 auf. Die untere Sitzbuchse 21 weist eine starre Stirnpartie 23 auf.

Der starre Dichtring 15 ist in Fig. 1 exemplarisch wellig, d. h. leicht gewölbt, ausgestaltet.

Die Aufnahmeeinrichtung 3 weist ferner einen teilweise halboffenen Strömungskanal 25 für die die Ventilvorrichtung 100 durchströmende Fluide, wie beispielsweise Blut und/oder Substituatflüssigkeit und/oder Sterilisierflüssigkeit, auf. Der Strömungskanal 25 weist Strömungskanalabschnitte 27 und 29 auf. In Fig. 1 ist, wie anhand der Pfeile angedeutet ist, der Strömungskanalabschnitt 27 als Einströmungskanal und der Strömungskanalabschnitt 29 als Ausströmungskanal gezeigt. Die Ventilvorrichtung 100 kann natürlich aber auch in einer anderen, insbesondere in umgekehrter Richtung durchströmt werden.

Der Ventileinsatz 1 kann den Strömungskanal 25 in Abhängigkeit des Funktionsmodus öffnen oder schließen, wobei der Ventileinsatz 1 entsprechend einen der vorstehend beschriebenen und auch im Folgenden anhand der Figuren dargestellten Ventilzustände einnimmt.

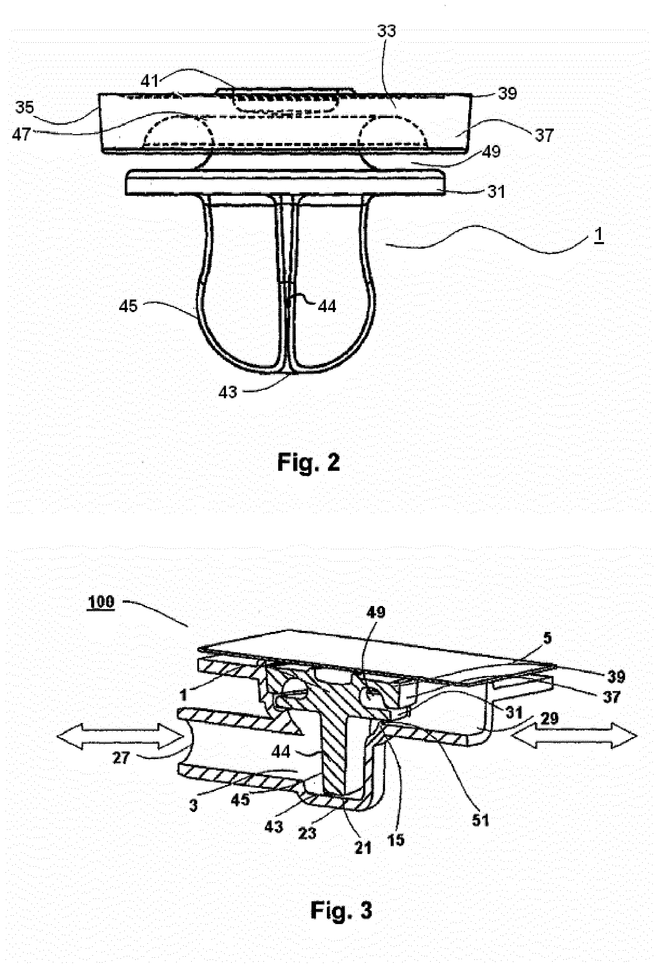

Fig. 2 zeigt eine schematische Seitenansicht eines teilweise geschnitten dargestellten Ventileinsatzes 1 einer offenbarten Ventilvorrichtung 100.

Der Ventileinsatz 1 weist eine erste Abdichtungseinrichtung, zum Beispiel in Gestalt eines elastischen Dichtrings 31, einen Biegeringbereich 33, sowie eine Führungseinrichtung, zum Beispiel in Form von Führungsrippen 45, auf. Ferner sind ein statischer Ventilsitz 35, ein elastischer Sitzbereich in Gestalt eines elastischen Sitzbereichs 37, ein äußerer Stirnringbereich 39, ein innerer Stirnringbereich 41 und ein Stirnanschlag 43 gezeigt.

Der elastische Dichtring 31 ist an seiner Unterseite (d. h. in der Darstellung der Fig. 2 unten) eben, d. h. flach, ausgestaltet.

Durch Aufbringen einer axialen Verschiebung und/oder einer axialen Kraft, insbesondere Verpresskraft auf den inneren Stirnringbereich 41 kann sich eine - z. B. axiale - Stauchzone 47 des Ventileinsatzes 1 elastisch verformen, z. B. zusammendrücken. Fig. 2 zeigt einen Verformungsraum 49, dessen Form und Volumen in Abhängigkeit des Ventilzustands der Ventilvorrichtung 100 variierbar sind.

Der o. g. zentrale Bereich ist in exemplarischer Form in Fig. 3 zu erkennen; er trägt dort das Bezugszeichen 44. Wie in Fig. 2 erkennbar ist, kann die Materialanhäufung des zentralen Bereichs 44 z. B. durch das Zusammenlaufen von Führungsrippen 45 in einer Symmetrieachse des Ventileinsatzes 1 entstehen.

Die Fig. 3 bis 7 zeigen verschiedene, exemplarisch ausgewählte Ventilzustände der Ventilvorrichtung 100, die durch Aufbringen einer Verschiebung und/oder einer Kraft, wie beispielsweise einer Verpresskraft seitens einer Behandlungsvorrichtung oder einer Kraft eines Fluids, auf den Ventileinsatz 1 erreichbar sind. Der Ventileinsatz 1 ist durch Aufbringen oder Aufheben der Kraft zwischen den Ventilzuständen schaltbar.

Fig. 3 zeigt schematisch vereinfacht einen Schnitt der Ventilvorrichtung 100 in einem Ventilzustand, welche im Folgenden als eine Sterilisations- oder Lagerungsstellung bezeichnet wird.

Der Ventileinsatz 1 ist in die Aufnahmeeinrichtung 3 eingelegt. Der Ventileinsatz 1 liegt mit seinen Führungsrippen 45 an der starren Stirnpartie 23 der unteren Sitzbuchse 21 der Aufnahmeeinrichtung 3 auf. Der Ventileinsatz 1 ist mit dem Folienelement 5 bedeckt.

Bei der Auslegung des Biegeringbereichs 33 des Ventileinsatzes 1 ist der Kraftschluss zwischen den Führungsrippen 45 (vgl. Fig. 2) und der Innenwandung der unteren Sitzbuchse 21 der Aufnahmeeinrichtung 3 bevorzugt nur so groß, dass ein Herausfallen des Ventileinsatzes 1 aufgrund seines Eigengewichtes vorteilhaft gerade sicher verhindert werden kann.

Der Biegeringbereich 33 ist bevorzugt so ausgelegt, dass eine vorübergehende, beispielsweise montagebedingte, axiale Verschiebung des Stirnanschlags 43 des Ventileinsatzes 1 bis an den Boden der unteren Sitzbuchse 21 durch die Rückstellkraft des Biegeringbereichs 33 vorteilhaft vollständig rückgängig gemacht wird. Die Rückstellkräfte des Biegeringbereichs 33 überwinden dabei bevorzugt die Reibungskräfte zwischen den Führungsrippen 45 und den Innenwandungen der unteren Sitzbuchse 21 oder Führungsbuchse der Aufnahmeeinrichtung 3. Auf diese Weise kann in vorteilhafter Weise eine besonders einfache Montage gewährleistet werden.

In der in Fig. 3 gezeigten Sterilisationsstellung wirkt keine äußere Kraft auf den Ventileinsatz 1. Der Ventileinsatz 1 ist damit im Wesentlichen keiner Vorspannung und/oder Materialbeanspruchung ausgesetzt. Alle hauptfunktionsrelevanten Bereiche des Ventileinsatzes 1 sind weder mit der Aufnahmeeinrichtung 3 noch mit dem Gehäuseelement 13 (hier nicht gezeigt) verpresst. Sie sind frei von relevanten Materialspannungen.

Da auch auf den elastischen Sitzbereich 37 in Fig. 3 keine Kraft oder Verschiebung einwirkt, nimmt der Verformungsraum 49 des Ventileinsatzes 1 ein maximales Volumen ein.

Die Ventilvorrichtung 100 befindet sich in einem für ein die Ventilvorrichtung 100 durchströmendes Fluid geöffneten Zustand. Dabei können die Fluide die Ventilvorrichtung 100 bidirektional durchströmen, wie anhand der in Fig. 3 dargestellten Doppelpfeile zu sehen ist

Die Fluide durchströmen die Ventilvorrichtung 100 durch einen Spalt 51, welcher sich zwischen dem elastischen Dichtring 31 des Ventileinsatzes 1 und dem starren Dichtring 15 der Aufnahmeeinrichtung 3 befindet.

Ein solcher Zustand eignet sich besonders zur Sterilisation der Ventilvorrichtung 100 oder zur Sterilisation einer mit einer Ventilvorrichtung 100 verbundenen externen Funktionseinrichtung.

Da die einzelnen Komponenten in einem solchen Ventilzustand spannungsfrei - sowohl zueinander als auch in sich - angeordnet sind, eignet sich dieser Ventilzustand ferner bevorzugt zum länger dauernden Lagern der Ventilvorrichtung 100 oder einer externen Funktionseinrichtung mit einer derartigen Ventilvorrichtung 100. Ein Spannungsverlust und eine damit verbundene Schädigung der betroffenen Bauteile treten mangels Spannung nicht auf

Durch die offene Sterilisationsstellung der Ventilvorrichtung 100 kann es nun möglich sein, sämtliche Sterilisations- oder sonstigen Vorbehandlungen durchzuführen, welche den freien bidirektionalen Durchtritt von Sterilisations- oder Behandlungsfluiden, wie beispielsweise Blut und/oder Substituatflüssigkeit, durch den Strömungskanal 25 erfordern.

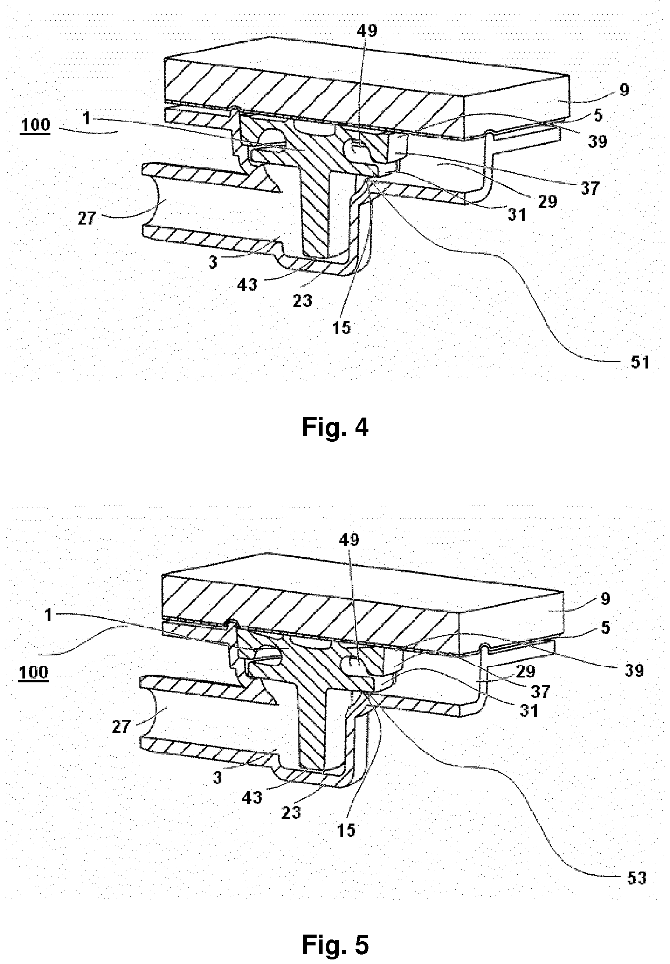

Fig. 4 zeigt schematisch vereinfacht eine seitliche Ansicht der Ventilvorrichtung 100 aus Fig. 3 in einer permanent geöffneten Ventilstellung als Ventilzustand. Im Gegensatz zur Darstellung der Fig. 3 ist die Ventilvorrichtung 100 in Fig. 4 in einer Behandlungsvorrichtung angeordnet. Dies ist daran zu erkennen, dass über dem Folienelement 5 ein Übertragungselement 9, beispielsweise ein Druckaktor einer Behandlungsvorrichtung, angeordnet ist.

Der Ventileinsatz 1 wird mittels der Führungsrippen 45 und über dem äußeren Stirnringbereich 39 zwischen der Aufnahmeeinrichtung 3 und dem Übertragungselement 9 gehalten.

Das Übertragungselement 9 kann so ausgelegt sein, dass der Ventileinsatz 1 in der permanent geöffneten Ventilstellung bidirektional durchströmbar ist. Die Ventilvorrichtung 100 befindet sich somit maschinenbedingt oder -gesteuert in geöffneter Position.

Das Übertragungselement 9 liegt auf dem Folienelement 5 derart auf, dass ein Abschluss zwischen Folienelement 5 und äußerem Stirnringbereich 39 erzielt ist, anders als in der in

Fig. 3 gezeigten Stellung, in welcher ein Raum zwischen Folienelement 5 und äußerem Stirnringbereich 39 zum Zweck des Sterilisierens offengehalten ist.

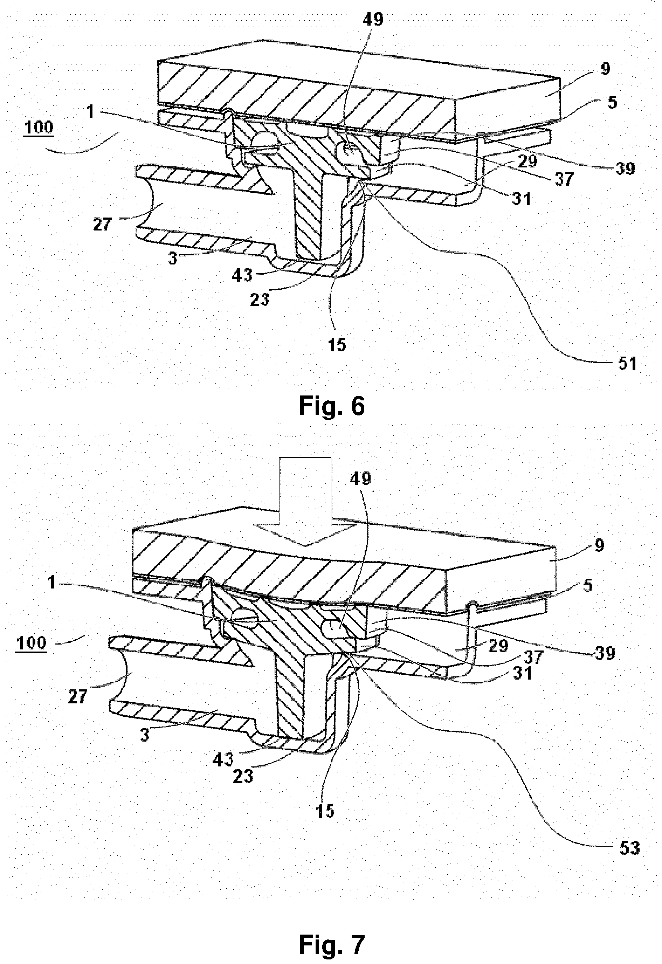

Fig. 5 und 6 zeigen schematisch vereinfacht seitliche Ansichten der Ventilvorrichtung 100 der Fig. 3 und 4 in einem Ventilzustand eines geschlossenen Rückschlagventils (Fig. 5) bzw. in einem Ventilzustand eines geöffneten Rückschlagventils (Fig. 6).

In Fig. 5 wird die Einbauverpressung des Ventileinsatzes 1 durch Verkürzen einer Weglänge (in axialer Richtung, also in "von oben nach unten"-Richtung in Fig. 5) zwischen der Aufnahmeeinrichtung 3 und dem Übertragungselement 9 erhöht und anschließend konstant gehalten. Dies kann bei passiven Maschinenanordnungen ohne aktive Ventilfunktionen allein durch das Einsetzen der externen Funktionseinrichtung in die Maschine geschehen. Bei aktiven Maschinenanordnungen mit zusätzlichen Auf-Zu-Funktionen der Ventilvorrichtungen 100 kann dies durch eine entsprechende Ansteuerung des Übertragungselementes 9 erfolgen.

Fig. 5 zeigt den vorgespannt geschlossenen Ventilzustand der Ventilvorrichtung 100, wenn ein anliegendes Fluid entgegen der Durchlassrichtung am starren Dichtring 15 ansteht, oder wenn das Fluid in Durchlassrichtung ansteht, die Druckdifferenz zwischen An- und Abströmseite aber noch kleiner ist als der eingestellte Mindestansprechdruck des Rückschlagventils.

In einem Berühr-/Verpressbereich 53 liegen der elastische Dichtring 31 und der starre Dichtring 15 aufeinander auf bzw. sind gegeneinander verpresst.

Fig. 6 zeigt den Ventilzustand während der Durchströmung der Ventilvorrichtung 100 in Durchlassrichtung. Vor allem in diesem Ventilzustand kann die zuvor beschriebene Toleranzausgleichsfunktion in axialer Richtung zwischen dem inneren Stirnringbereich 41 und dem Stirnanschlag 43 erfolgen und sorgt für konstante Funktionseigenschaften. Dabei befindet sich der Stirnanschlag 43 des Ventileinsatzes 1 in der Rückschlagventilstellung bereits im Anschlag an der starren Stirnpartie 23 der unteren Sitzbuchse 21.

Zwischen dem elastischen Dichtring 31 und dem starren Dichtring 15 verbleibt ein Spalt 51.

Fig. 7 zeigt schematisch vereinfacht eine seitliche Ansicht einer Ventilvorrichtung 100 in einem Ventilzustand als permanent geschlossenes Ventil oder als Regelventil. Ein solches Regelventil kann als Druck- oder Volumenstromregelventil fungieren.

In diesem Ventilzustand erhöht das Übertragungselement 9 die Verpressung in axialer Richtung (gemeint ist eine Richtung, welche sich in der Figur senkrecht von oben nach unten erstreckt) durch ein weiteres Stück axiale Verschiebung so weit, bis der äußere Stirnringbereich 39 in mechanische Verpressung mit dem elastischen Dichtring 31 in der axialen Richtung gerät.

Auf diese Weise wird die Verpressung zwischen dem elastischen Dichtring 31 und dem starren Dichtring 15 so weit gesteigert, dass die Ventilvorrichtung 100 bidirektional gegen die Auslegungsdruckdifferenz abgeschlossen wird.

In dem in Fig. 7 gezeigten Ventilzustand kann die offenbarte Ventilvorrichtung 100 als Druck- oder Volumenstromregelventil fungieren.

Wenn der in axialer Richtung elastische Sitzbereich 37 des Ventileinsatzes 1 oder der als Berührpartner zugeordnete elastische Dichtring 31 in definierter Weise nachgiebig gestaltet sind, etwa indem man ihn oder sie mit einer zusätzlichen Nut oder mit nachgiebigen dünnen Ringstegen oder Noppen versieht, welche bei der oben genannten weiteren axialen Auslenkung des Ventileinsatzes 1 eine gewollte Kontaktaufnahme zwischen dem axialen elastischen Sitzbereich 37 und dem elastischen Dichtring 31 zur Folge haben, dann kann es in diesem Ventilzustand zu einer definiert höheren Vorspannung der Ventilvorrichtung 100 kommen.

Eine derartige höhere Vorspannung kann entweder in zwei diskreten Stufen (Spalt 51 zwischen axialem elastischen Sitzbereich 37 und elastischem Dichtring 31) vor axialer Betätigung und Berührung nach axialer Betätigung oder in kontinuierlicher Weise (anfängliche Berührung zwischen den genannten Partnern) wegabhängig von der axialen Verschiebung des Übertragungselementes 9 erfolgen.

Fig. 8 zeigt eine Seitenansicht einer externen Funktionseinrichtung, welche an der Oberfläche, auf die in Fig. 8 geblickt wird, mit einer Abdeckungseinrichtung versehen ist.

Die externe Funktionseinrichtung ist hier exemplarisch als Blutbehandlungskassette 1000 ausgestaltet mit Kammern, Kanälen, Ventilen und dergleichen. Die Blutbehandlungskassette 1000 der Fig. 8 ist an ihrer Vorderseite mit einer Abdeckungseinrichtung, hier beispielsweise einer wie oben bereits eingeführten Folie 5, abgedeckt.

Die Blutbehandlungskassette 1000 kann wenigstens mit der in Fig. 8 gezeigten Vorderseite an eine Blutbehandlungsvorrichtung (in Fig. 1 nicht gezeigt) angekoppelt werden.

Die Blutbehandlungskassette 1000 weist eine arterielle Heparin-Zugabestelle 63 auf. Dabei gilt zu beachten, dass die Heparin-Zugabestelle 63 auch zur Zugabe anderer pharmakologischer Wirkstoffe als Heparin, welche nur vorzugsweise Antikoagulantien sind, oder Wirkstoffkombinationen geeignet und vorgesehen sein kann. Dies ist stets auch dann zu beachten, wenn zuvor oder im Folgenden von Heparin in beliebigem Zusammenhang die Rede ist.

Die Blutbehandlungskassette 1000 weist ein Rückschlagventil 65 der arteriellen Heparin-Zugabestelle 63 auf. Das Rückschlagventil 65 ist ein Anwendungsbeispiel der offenbarten Ventilvorrichtung der vorliegenden Anmeldung.

Die Blutbehandlungskassette 1000 weist ferner ein Rückschlagventil 69 für eine venöse Heparin-Zugabestelle 67 auf.

Über Betätigung eines Rückschlagventils 71 als weitere Ausgestaltungsform der offenbarten Ventileinrichtung kann Substituat in eine Substituatleitung 73 eingebracht werden.

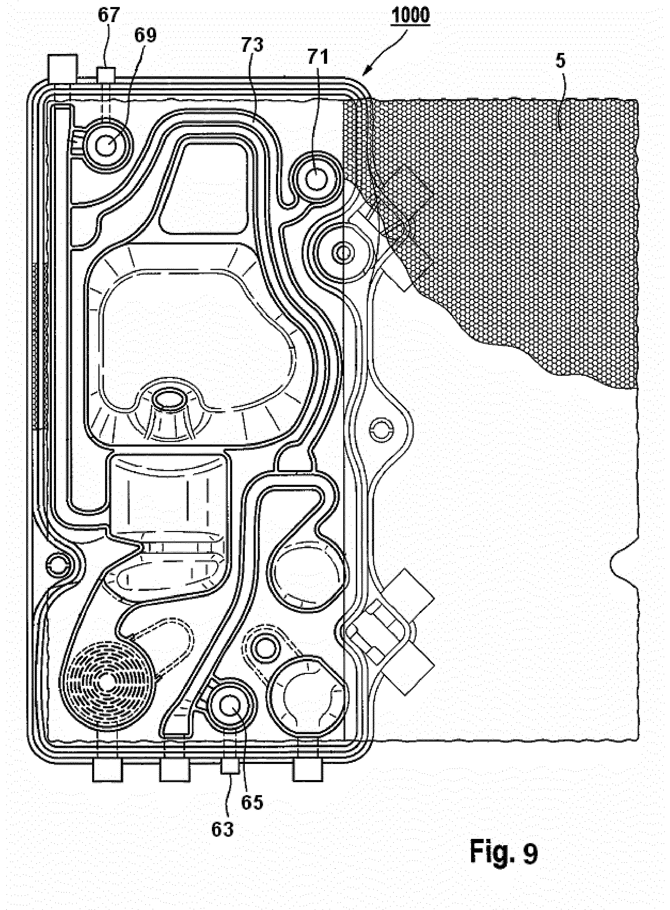

**Fig. 9** zeigt die Blutbehandlungskassette 1000 der Fig. 8, wobei die Folie am linken Rand der Blutbehandlungskassette 1000 sowie oben und unten ausgeschnitten und nach rechts aufgeklappt zu erkennen ist.

Fig. 9 zeigt die nach Aufschneiden der Folie detaillierter zu erkennenden Elemente im Inneren der Blutbehandlungskassette 1000.

Zur Vermeidung von Wiederholungen wird auf die vorstehend bei der Beschreibung der Fig. 8 erörterten Ausgestaltungen der einzelnen Elemente verwiesen.

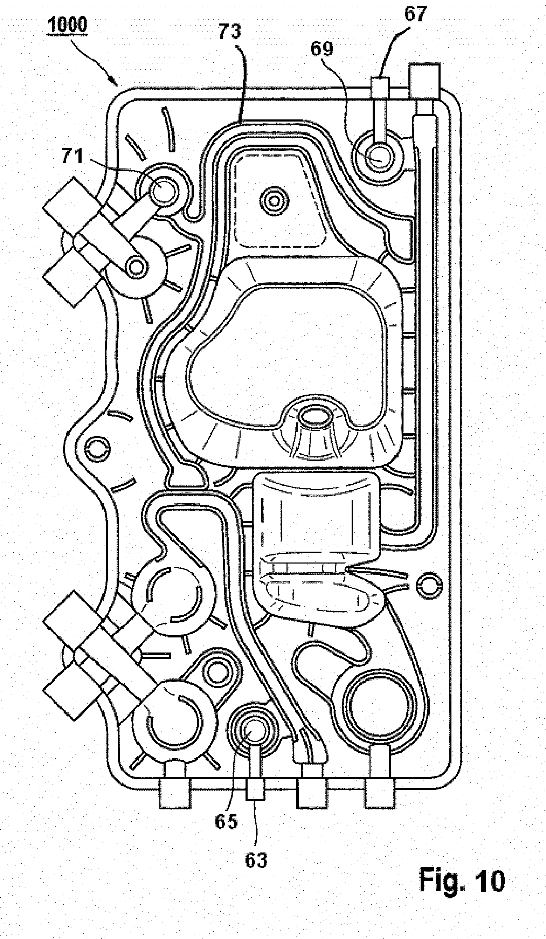

Fig. 10 zeigt die Blutbehandlungskassette 1000 von ihrer Rückseite. Ist die Blutbehandlungskassette 1000 an die Blutbehandlungsvorrichtung angekoppelt, so wird ein Betrachter beim Öffnen einer Tür der Blutbehandlungsvorrichtung zum Entnehmen der Blutbehandlungskassette 1000 auf diese Rückseite blicken.

Für weitere Details zur Blutbehandlungskassette 1000 wird auf deren ausführliche Beschreibung in der hierauf gerichteten, o. g. Anmeldung verwiesen.

## Patentansprüche

1. Externe Funktionseinrichtung, welche wenigstens ein Gehäuseelement (13) mit wenigstens einem Strömungskanal (25) und eine Ventilvorrichtung (100) aufweist, wobei die externe Funktionseinrichtung ausgestaltet ist als medizinische Fluidbehandlungskassette, wobei die Ventilvorrichtung (100) aufweist:
- wenigstens einen in wenigstens einem Abschnitt hiervon elastischen Ventileinsatz (1), welcher angeordnet und ausgestaltet ist, den Strömungskanal (25) zu öffnen oder zu schließen, wobei der Ventileinsatz (1) einen elastischen Biegeringbereich (33), einen elastischen Dichtring (31) als eine erste Abdichtungseinrichtung und eine Führungseinrichtung aufweist;
- wenigstens eine Aufnahmeeinrichtung (3) für den Ventileinsatz (1), wobei die Aufnahmeeinrichtung (3) in dem Gehäuseelement (13) vorgesehen ist und eine zweite Abdichtungseinrichtung (15) aufweist, wobei der Ventileinsatz (1) in der Aufnahmeeinrichtung (3) angeordnet ist, um mittels Kraftübertragung und/oder Wegeinprägung auf den Ventileinsatz (1) mittels des elastischen Dichtrings (31) form- und/oder kraftschlüssig gegen die zweite Abdichtungseinrichtung (15) verpressbar zu sein;
wobei der Ventileinsatz (1) derart ausgestaltet und im Strömungskanal (25) angeordnet ist, dass er aufgrund seiner Elastizität bei der Wegeinprägung und/oder Kraftaufbringung zwischen wenigstens drei unterschiedlichen Ventilzuständen schaltbar ist, wobei in einem Ventilzustand keine äußere Kraft oder keine oder keine wesentliche Vorspannung auf den Ventileinsatz (1) wirkt, wobei dieser Ventilzustand einer bidirektionalen Durchfluss-Stellung durch den Strömungskanal (25) entspricht.

2. Externe Funktionseinrichtung nach Anspruch 1, wobei einer der weiteren Ventilzustände ein geöffneter Funktionszustand und/oder ein Rückschlag-Ventilzustand und/oder ein Steuer- oder Regel-Ventilzustand und/oder ein geschlossener Ventilzustand des Ventils und/oder ein Sterilisationszustand ist.

3. Externe Funktionseinrichtung nach einem der vorangegangenen Ansprüche, wobei der Ventileinsatz (1) wenigstens in einem Abschnitt ein elastomeres Material aufweist.

4. Externe Funktionseinrichtung nach einem der vorangegangenen Ansprüche, wobei der Ventileinsatz (1) wenigstens in einem Abschnitt Einprägungen aufweist.

5. Externe Funktionseinrichtung nach einem der vorangegangenen Ansprüche, wobei die Kraft auf den Ventileinsatz (1) eine Verpresskraft ist, die durch Einbau der Ventilvorrichtung (100) in eine Behandlungsvorrichtung einleitbar ist.

6. Externe Funktionseinrichtung nach einem der vorangegangenen Ansprüche, wobei die Wegeinprägung auf den Ventileinsatz (1) eine Verschiebung ist, die durch Einbau der Ventilvorrichtung (100) in eine Behandlungsvorrichtung einleitbar ist.

7. Externe Funktionseinrichtung nach Anspruch 6, wobei die Verpresskraft oder die Verschiebung durch ein Übertragungselement (9) der Behandlungsvorrichtung übertragbar ist.

8. Externe Funktionseinrichtung nach einem der vorangegangenen Ansprüche, wobei die Ventilvorrichtung (100) an wenigstens einer Oberseite mit einer Abdeckungseinrichtung versehen ist.

9. Externe Funktionseinrichtung nach Anspruch 8, wobei der Ventileinsatz (1) mittels einer von einer Behandlungsvorrichtung übertragenen Kraft und/oder Wegeinprägung durch die Abdeckungseinrichtung hindurch schaltbar ist.

10. Behandlungsvorrichtung, welche wenigstens eine externe Funktionseinrichtung gemäß einem der Ansprüche 1 bis 9 aufweist, wobei die Behandlungsvorrichtung als Blutbehandlungsvorrichtung ausgestaltet ist.

## Claims

1. An external functional device which comprises at least one housing element (13) having at least one flow channel (25) and one valve device (100), the external functional device being designed as a medical fluid treatment cassette,
the valve device (100) comprising:
- at least one valve insert (1) elastic in at least one section thereof, which is arranged and designed to open or close the flow channel (25), the valve insert (1) comprising an elastic bending ring area (33), an elastic sealing ring (31) as a first sealing device and a guiding device,
- at least one receiving device (3) for the valve insert (1), the receiving device (3) being provided in the housing element (13) and comprising a second sealing device (15), the valve insert (1) being arranged in the receiving device (3) in order to be form-locking and/or force-locking compressible against the second sealing device (15) by means of force transmission and/or displacement imprint on the valve insert (1) by means of the elastic sealing ring (31);
wherein the valve insert (1) is designed and arranged in the flow channel (25) in such a way that, due to its elasticity during displacement imprint and/or pressing force, it can be switched between at least three different valve states, wherein in a valve state, no external force or no preload or no significant preload acts on the valve insert (1), this valve state corresponding to a bidirectional flow position through the flow channel (25).

2. The external functional device according to claim 1, wherein one of the further valve states is an open functional state and/or a check valve state and/or a control or regulating valve state and/or a closed valve state of the valve and/or a sterilization state.

3. The external functional device according to anyone of the preceding claims, wherein the valve insert (1) comprises an elastomeric material in at least one section.

4. The external functional device according to anyone of the preceding claims, wherein the valve insert (1) comprises imprints in at least one section.

5. The external functional device according to anyone of the preceding claims, wherein the force on the valve insert (1) is a compression force which can be initiated by installing the valve device (100) in a treatment apparatus.

6. The external functional device according to anyone of the preceding claims, wherein the displacement imprint on the valve insert (1) is a shift which can be initiated by installing the valve device (100) in a treatment apparatus.

7. The external functional device according to claim 6, wherein the compression force or the shift can be transmitted by a transmission element (9) of the treatment apparatus.

8. The external functional device according to anyone of the preceding claims, wherein the valve device (100) is provided on at least one top side with a cover device.

9. The external functional device according to claim 8, wherein the valve insert (1) can be switched by means of a force transmitted by a treatment device and/or by a displacement imprint throughout the cover device.

10. A treatment apparatus which comprises at least an external functional device according to anyone of claims 1 to 9, wherein the treatment apparatus is designed as a blood treatment apparatus.

## Revendications

1. Un dispositif fonctionnel externe qui comprend au moins un élément de boîtier (13) ayant au moins un canal d'écoulement (25) ainsi qu'un dispositif de vanne (100), le dispositif fonctionnel externe étant conçu comme une cassette de traitement de fluide médicale, le dispositif de vanne (100) comprenant:
- au moins un pointeau (1) élastique dans au moins une section de celui-ci, agencé et conçu de façon à ouvrir et fermer le canal d'écoulement (25), le pointeau (1) comprenant une zone d'anneau de flexion élastique (33), un anneau d'étanchéité (31) en tant que premier dispositif d'étanchéité ainsi qu'un dispositif de guidage,
- au moins un dispositif de réception (3) pour le pointeau (1), le dispositif de réception (3) étant prévu dans l'élément de boîtier (13) et comprenant un second dispositif d'étanchéité (15), le pointeau (1) étant agencé dans le dispositif de réception (3) afin d'être compressible en complémentarité de forme et/ou de force contre le second dispositif d'étanchéité (15) par transmission de force et/ou d'empreinte par déplacement sur le pointeau (1) au moyen de l'anneau d'étanchéité élastique (31);
où le pointeau (1) est conçu et agencé dans le canal d'écoulement (25) de telle sorte qu'en raison de son élasticité lors de l'empreinte par déplacement et/ou de l'application de force, il soit commutable entre au moins trois états de vanne différents, où dans un état de vanne, aucune force extérieure ou aucune pré-tension ou aucune pré-tension significative n'agit sur le pointeau (1), cet état de vanne correspondant à une position d'écoulement bidirectionnelle au travers du canal d'écoulement (25).

2. Le dispositif fonctionnel externe selon la première revendication, où un des autres états de vanne est un état fonctionnel ouvert et/ou un état de vanne anti-retour et/ou un état de vanne de commande ou de régulation et/ou un état de vanne fermé de la vanne et/ou un état de stérilisation.

3. Le dispositif fonctionnel externe selon l'une quelconque des revendications précédentes, où le pointeau (1) comprend un matériau élastomère dans au moins une section.

4. Le dispositif fonctionnel externe selon l'une quelconque des revendications précédentes, où le pointeau (1) comprend des empreintes dans au moins une section.

5. Le dispositif fonctionnel externe selon l'une quelconque des revendications précédentes, où la force exercée sur le pointeau (1) est une force de compression pouvant être initiée par l'installation du dispositif de vanne (100) dans un appareil de traitement.

6. Le dispositif fonctionnel externe selon l'une quelconque des revendications précédentes, où l'empreinte par déplacement effectuée sur le pointeau (1) est un décalage pouvant être initié par l'installation du dispositif de vanne (100) dans un appareil de traitement.

7. Le dispositif fonctionnel externe selon la revendication 6, où la force de compression ou le décalage peut être transmis par un élément de transmission (9) de l'appareil de traitement.

8. Le dispositif fonctionnel externe selon l'une quelconque des revendications précédentes, où le dispositif de vanne (100) est muni d'un dispositif de recouvrement sur au moins une face supérieure.

9. Le dispositif fonctionnel externe selon la revendication 8, où le pointeau (1) peut être commuté au moyen d'une force transmise(s) par un appareil de traitement et/ou d'une empreinte par déplacement au travers du dispositif de recouvrement.

10. Un appareil de traitement qui comprend au moins un dispositif fonctionnel externe selon une des revendications 1 à 9, où l'appareil de traitement est conçu sous la forme d'un appareil de traitement du sang.
